(19) Europäisches Patentamt
European Patent Office
Office européen des brevets

(11) Veröffentlichungsnummer: **0 002 822**
**B1**

(12) # EUROPÄISCHE PATENTSCHRIFT

(45) Veröffentlichungstag der Patentschrift: **28.07.82**

(51) Int. Cl.³: **C 07 F 9/38**, A 61 K 37/02

(21) Anmeldenummer: **78101826.2**

(22) Anmeldetag: **22.12.78**

(54) **Phosphorhaltige Peptidderivate, deren Herstellung, Zwischenprodukte bei deren Herstellung und diese enthaltende pharmazeutische Präparate.**

(30) Priorität: **23.12.77 GB 5366877**
**20.11.78 GB 4527278**

(43) Veröffentlichungstag der Anmeldung:
**11.07.79 Patentblatt 79/14**

(45) Bekanntmachung des Hinweises auf die Patenterteilung:
**28.07.82 Patentblatt 82/30**

(84) Benannte Vertragsstaaten:
**BE CH DE FR IT LU NL SE**

(56) Entgegenhaltungen:
**DE - A - 2 602 193**
**DE - A - 2 721 760**
**DE - A - 2 721 761**
**DE - A - 2 730 524**

**F. Klages, Lehrbuch der organischen Chemie, Bd. III (1958) s. 416—419**
**Schröder et al, Arzneimittelchemie I (1976), s. 24—25 Fieser/Fieser, Organische Chemie (1968) s. 1261**

Die Akte enthält technische Angaben die nach dem Eingang der Anmeldung eingereicht wurden und die nicht in dieser Patentschrift enthalten sind.

(73) Patentinhaber: **F. HOFFMANN-LA ROCHE & CO. Aktiengesellschaft**
**CH-4002 Basel (CH)**

(72) Erfinder: **Atherton, Frank Ratcliffe**
**148 Parkway**
**Welwyn Garden City Herts. (GB)**
Erfinder: **Hall, Michael John**
**15 Cannonsfield Road**
**Welwyn Herts. (GB)**
Erfinder: **Hassall, Cedric Herbert**
**"Trelech" Tewin Close Tewin Wood**
**Welwyn Herts. (GB)**
Erfinder: **Lambert, Robert Wilson**
**6 Grange Hill**
**Welwyn Herts. (GB)**
Erfinder: **Ringrose, Peter Stuart**
**41 The Limes**
**Harston Cambridgeshire (GB)**

(74) Vertreter: **Lederer, Franz, Dr. et al,**
**Patentanwälte Lederer, Meyer Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 822 B1

# Phosphorhaltige Peptidderivate deren Herstellung, Zwischenprodukte bei deren Herstellung und diese enthaltende pharmazeutische Präparate

Die erfindungsgemässen Peptidderivate sind Verbindungen der allgemeinen Formel

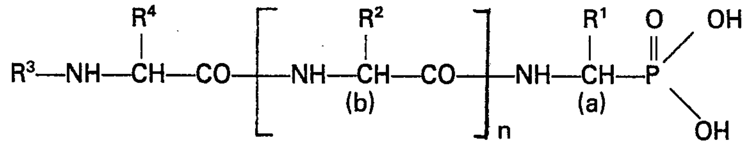

(i)

worin $R^1$ Wasserstoff oder Methyl; $R^2$ eine für $\alpha$-Aminosäuren, wie sie normalerweise in Proteinen verkommen, charakteristische Gruppe, oder eine von den Alkyl- bzw. Hydroxyalkyl-gruppen, wie sie für normalerwise in Proteinen vorkommende $\alpha$-Aminosäuren charakteristisch sind, sich unterscheidende $C_2$—$C_8$-Alkyl- bzw. $C_2$—$C_8$-Hydroxyalkylgruppe; $R^3$ $C_1$—$C_8$-Alkyl, $C_3$—$C_6$-Cycloalkyl oder Allyl; und $R^4$ Wasserstoff oder $C_1$—$C_8$-Alkyl bedeuten; n für 2 oder 3 steht; mit R-Konfiguration am mit (a) bezeichneten C-Atom (falls $R^1 \neq H$) und Konfiguration am mit (b) bezeichneten C-Atom (falls $R^2 \neq H$); sowie pharmazeutisch verträgliche Salze solcher Verbindungen.

Die Bezeichnung $C_1$—$C_8$-bzw. $C_2$—$C_8$-Alkyl soll im vorliegenden Zusammenhang eine gerad- oder verzweigtkettige Alkylgruppe mit 1—8 bzw. 2—8 C-Atomen bedeuten, z.B. Methyl, Aethyl, Propyl, Isopropyl, Butyl, tert.Butyl, Pentyl, Hexyl. Beispiele von $C_2$—$C_8$-Hydroxyalkylgruppen sind 2-Hydroxyäthyl, 3-Hydroxypropyl, 4-Hydroxybutyl. Beispiele für $C_3$—$C_6$-Cycloalkylgruppen sind Cyclopropyl und Cyclobutyl. Die Bezeichnung "für $\alpha$-Aminosäuren, wie sie normalerweise in Proteinen vorkommen, charakteristische Gruppe" bedeutet den Rest R einer natürlichen $\alpha$-Aminosäure der allgemeinen Formel

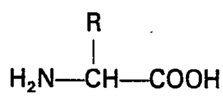

wie sie normalerweise in Proteinen vorkommen. So bedeutet R, falls die $\alpha$-Aminosäure Glycin ist, Wasserstoff, falls die $\alpha$-Aminosäure Alanin ist, Methyl, im Fall von Methionin 2-Methylthioäthyl, im Fall von Serin Hydroxymethyl und im Fall von Tyrosin p-Hydroxybenzyl. R kann auch zusammen mit dem Stickstoff der Aminogruppe zum Ring geschlossen sein, wie in Prolin. Die normalerweise in Proteinen vorkommenden Aminosäuren sind in Fieser/Fieser, Organische Chemie, Verlag Chemie, Weinheim, 1968, S. 1261, zusammengestellt.

Wenn $R^1$ in der Formel I nicht Wasserstoff darstellt, dann soll die Konfiguration am mit (a) bezeichneten C-Atom (R) sein, d.h. die Konfiguration soll so sein, wie sie beim Ersatz der Carboxylgruppe einer Natürlichen $\alpha$-Aminosäure durch eine $PO_3H_2$-Gruppe erhalten würde.

$R^2$ kann gleiche oder verschiedene Reste bedeuten.

Bevorzugte erfindungsgemässe Peptidderivate sind solche, in denen $R^1$ Methyl bedeutet sowie diejenigen, in denen $R^2$ eine für $\alpha$-Aminosäuren, wie sie normalerweise in Proteinen vorkommen, charakteristische Gruppe oder eine sich von solchen, charakteristisch en Gruppen unterscheidende $C_1$—$C_8$-Alkylgruppe darstellt. Ebenfalls bevorzugt sind solche Peptidderivate, in welchen $R^3$ für $C_1$—$C_8$-Alkyl, insbesondere Methyl steht.

Beispiele von Verbindungen der Formel I sind:
(1R)-1-(Sarcosyl-L-alanyl-L-alanylamino)äthylphosphonsäure,
(1R)-1-(SarcosylL-methionyl-L-alanylamino)-äthylphosphonsäure,
(1R)-1-(Sarcosyl-L-histidyl-L-alanylamino)-äthylphosphonsäure,
(1R)-1-(Sarcosyl-L-tyrosyl-L-alanylamino)-äthylphosphonsäure,
(1R)-1-(Sarcosyl-L-alanyl-L-arginylamino)-äthylphosphonsäure,
(1R)-1-(Sarcosyl-L-alanyl-L-serylamino)-äthylphosphonsäure,
(Sarcosyl-L-alanyl-L-alanylamino)-methylphosphonsäure,
(1R)-1-(Sarcosyl-L-norvalyl-L-norvalylamino)-äthylphosphonsäure,
(1R)-1-(Sarcosyl-glycyl-L-norvalyl-L-norvalylamino)-äthylphosphonsäure,
(Sarcosyl-L-norvalyl-L-norvalylamino)-methylphosphonsäure,
(1R)-1-(N-Methyl-L-norvalyl-L-norvalyl-L-norvalylamino)-äthylphosphonsäure,
(1R)-1-(N-Aethyl-glycyl-L-alanyl-L-alanylamino)-äthylphosphonsäure,
(1R)-1-(N-(n-Propyl)-glycyl-L-alanyl-L-alanylamino)-äthylphosphonsäure,
(1R)-1-(N-Allyl-glycyl-L-alanyl-L-alanylamino)-äthylphosphonsäure,
(1R)-1-(N-(n-Hexyl)-glycyl-L-alanyl-L-alanylamino)-äthylphosphonsäure,
(1R)-1-(N-Cyclopropyl-glycyl-L-alanyl-L-alanylamino)-äthylphosphonsäure,
(1R)-1-(N-tert.Butyl-glycyl-L-alanyl-L-alanylamino)-äthylphosphonsäure,
(1R)-1-(N-Methyl-L-valyl-L-valyl-L-norvalylamino)-äthylphosphonsäure,
(1R)-1-(N-Methyl-L-leucyl-L-norvalyl-L-norvalylamino)-äthylphosphonsäure,
(1R)-1-(Sarcosyl-L-valyl-L-valyl-L-norvalylamino)-äthylphosphonsäure,
(1R)-1-(N-Methyl-L-valyl-L-norvalyl-L-norvalylamino)-äthylphosphonsäure.

Die erfindungsgemässen Peptidderivate können dadurch erhalten werden, dass man
a) die Schutzgruppe (n) aus einer Verbindung der allgemeinen Formel

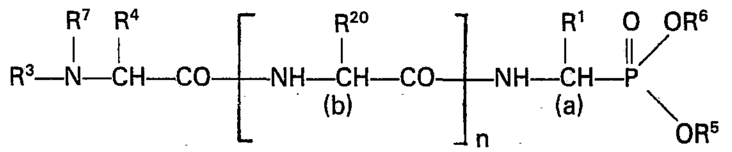

(Ia)

worin n, $R^1$, $R^3$ und $R^4$ dasselbe wie oben $R^5$ und $R^6$ Wasserstoff oder Niederalkylschutzgruppen, $R^7$ eine Aminoschutzgruppe und $R^{20}$ dasselbe wie $R^2$, bedenten wobei jede Aminogruppe geschützt sein kann und gegebenenfalls vorhandene andere funktionelle Gruppen erforderlichenfalls geschützt sein können und worin (a) und (b) wie oben definiert sind, nach an sich bekannten Methoden abspaltet und

b) eine erhaltene Verbindung der allgemeinen Formel I gewünschtenfalls in ein pharmazeutisch verträgliches Salz überführt.

Die Verbindungen der Formel Ia können dadurch hergestellt werden, dass man eine Verbindung der allgemeinen Formel

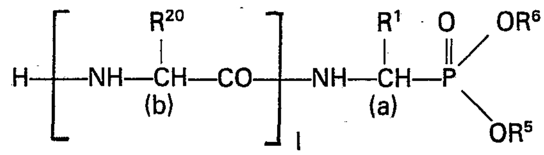

(II)

mit einer Verbindung der allgemeinen Formel

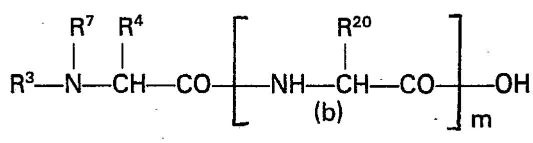

(III)

kondensiert, wobei l und m für 0, 1, 2 oder 3 stehen, mit der Massgabe, dass die Summe von l und m 2 oder 3 ist, und $R^1$, $R^3$, $R^4$, $R^5$, $R^6$, $R^7$ und $R^{20}$ dasselbe wie oben bedeuten.

Die Schutzgruppe $R^7$ kann eine jede aus der Peptidchemie wohlbekannte Aminoschutzgruppe sein. In einer bevorzugten Ausführungsart des erfindungsgemässen Verfahrens ist diese Aminoschutzgruppe eine Aralkoxycarbonylgruppe, besonders Benzyloxycarbonyl, oder tert. Butoxycarbonyl. Die Aminoschutzgruppe kann jedoch z.B. auch eine Formyl-, Trityl-, Trifluoracetyl- oder die 2-(Biphenylyl)-isopropyloxycarbonylgruppe sein. Eine gegebenenfalls in $R^{20}$ vorliegende Aminogruppe kann auf entsprechende Art geschützt sein. Eine in $R^{20}$ anwesende Hydroxy=gruppe kann geschützt sein durch eine übliche Hydroxyschutzgruppe, z.B. Aralkoxycarbonyl, wie Benzyloxycarbonyl; Niederalkanoyl, wie Acetyl, Propionyl; Aroyl, wie z.B. Benzoyl; Niederalkyl, wie z.B. tert.Butyl; oder Niederaralkyl, wie z.B. Benzyl. Jede gegebenenfalls in $R^{20}$ anwesende Carboxygruppe kann mit einer üblichen Carboxyschutzgruppe geschützt sein. So kann eine Carboxygruppe z.B. durch Ueberführung in einen Alkylester, z.B. den tert.Butylester, oder in einen Aralkylester, z.B. den Benzylester, geschützt werden. Andere in $R^{20}$ gegebenenfalls anwesende funktionelle Gruppen können auf an sich bekannte Art geschützt werden.

Die Kondensation einer Verbindung II mit einer Verbindung III kann nach in der Peptidchemie an sich bekannten Methoden durchgeführt werden, z.B. nach der gemischten Anhydrid-, Azid-, aktivierte Ester-, Säurechlorid-, Carbodiimid- oder EEDQ-Methode (EEDQ=1-Aethoxycarbonyl-2-äthoxy-1,2-dihydrochinolin). In einer bevorzugten Ausführungsart kondensiert man eine Verbindung II, worin l für 2 oder 3 steht, mit einer Verbindung III, worin m für 0 steht.

Nach einer Methode kann man eine Verbindung II mit einer Verbindung III in Form eines gemischten Anhydrids mit einer organischen oder anorganischen Säure kondensieren. Zweckmässigerweise wird eine Verbindung III mit einer tertiären Base, wie einem Tri-niederalkylamin, z.B. Triäthylamin, oder N-Aethylmorpholin, in einem inerten organischen Lösungsmittel, z.B. Tetrahydrofuran, 1,2-Dimethoxyäthan, Dichlormethan, Toluol, Petroläther, behandelt und das erhaltene Salz bei niedriger Temperatur mit einem geeigneten Chlorformiat, z.B. einem Niederalkylchlorformiat, wie dem Aethylchlorformiat, oder dem Isobutylchlorformiat, umgesetzt. Das so erhaltene gemischte Anhydrid wird dann zweckmässigerweise in situ mit einer Verbindung II umgesetzt.

Nach einer anderen Methode kann eine Verbindung II mit einer Verbindung III kondensiert werden, in welche letzterer die Carboxygruppe in eine Säureazidgruppe umgewandelt worden ist. Diese Kondensation wird vorzugsweise in einem inerten organischen Lösungsmittel, wie Dimethylformamid oder Aethylacetat bei niedriger Temperatur ausgeführt.

Nach einer weiteren Methode kann eine Verbindung II mit einer Verbindung III kondensiert werden, in welche letzterer die Carboxygruppe in Form einer aktiven Estergruppe vorliegt, z.B. der 4-Nitrophenyl-, 2,4,5-Trichlorphenyl- oder der N-Hydroxysuccinimidestergruppe. Diese Kondensation

wird zweckmässigerweise in einem inerten Lösungsmittel, wie wässrigem Dimethylformamid, oder, falls sowohl $R^5$ wie $R^6$ Niederalkyl bedeuten, auch in einem niedern Alkanol, wie Aethanol, durchgeführt.

Nach einer anderen Methode kann eine Verbindung II mit einer Verbindung III kondensiert werden, in welche letzterer die Carboxygruppe in Form eines Säurechlorids vorliegt. Diese Kondensation erfolgt zweckmässigerweise in Gegenwart einer Base und niedriger Temperatur, vorzugsweise in einem nichthydrolytischen Lösungsmittel.

Schliesslich kann man eine Verbindung II mit einer Verbindung III in Gegenwart eines Carbodiimids, z.B. Dicyclohexylcarbodiimid oder EEDQ, kondensieren. Diese Kondensation kann in einem inerten organischen Lösungsmittel, z.B. Methylenchlorid oder einem niedern Alkanol, wie Methanol, Aethanol, bei Raumtemperatur oder darunter, durchgeführt werden.

Die Kondensation einer nichtgeschützen Verbindung Ia mit einer Verbindung III kann auf ähnliche Art wie für die Kondensation der Verbindungen II und III beschrieben, durchgeführt werden.

Die Abspaltung von Schutzgruppen aus dem Kondensationsprodukt kann nach an sich bekannten Methoden bewerkstelligt werden, d.h. nach Methoden, wie sie in der Literatur beschrieben bzw. in Gebrauch sind. So kann z.B. eine Aralkoxycarbonylgruppe, wie Benzyloxycarbonyl, die tert.Butoxycarbonylgruppe oder die 2 - Biphenylyl - isopropyloxycarbonylgruppe durch Hydrolyse abgespalten werden, z.B. durch Behandlung mit HBr in Eisessig. Eine Aralkoxycarbonylgruppe, z.B. Benzyloxycarbonyl, kann auch mittels Hydrogenolyse abgespalten werden, z.B. in Gegenwart von Palladiumkohle-Katalysator oder Platinoxyd. Die tert.Butoxycarbonyl- oder 2 - Biphenylyl - isopropyloxycarbonylgruppe können auch mit HCl in Dioxan abgespalten werden. Eine Tritylgruppe kann z.B. durch Behandlung mit verdünnter Essigsäure abgespalten werden. Wenn das Kondensationsprodukt eine 2 - Methylthioäthylgruppe als $R^{20}$-Gruppe und eine Aralkoxycarbonylschutzgruppe, z.B. Benzyloxycarbonyl, enthält, dann erfolgt die Abspaltung der genannten Schutzgruppe vorzugsweise in Gegenwart von Diäthylphosphit oder Methyläthylsulfid. Niedere Alkylschutzgruppen $R^5$ und $R^6$ können durch Behandlung mit HBr in Eisessig oder unter Verwendung von Trimethylchlorsilan oder Trimethylbromsilan gefolgt von wässriger Hydrolyse, abgespalten werden. Es versteht sich, dass bei Anwesenheit von mehr als einer Schutzgruppe die Abspaltung je nach der Natur besagter Gruppen in einem oder mehreren Schritten erfolgen kann. Vorzugsweise wird man jedoch Schutzgruppen verwenden, welche sich in einem einzigen Schritt abspalten lassen.

Die Verbindungen der Formel I sind amphoter und bilden demgemäss Salze sowohl mit Säuren wie mit Basen. Zur Bildung pharmazeutisch verträglicher Salze eignen sich starke Säuren, wie z.B. Salzsäure, Bromwasserstoff, Schwefelsäure, Methansulfonsäure, p-Toluolsulfonsäure, oder Basen, wie z.B. Natrium- oder Kaliumhydroxyd.

Die Verbindungen der Formel II, worin l für 0 steht, sind bekannt bzw. können in Analogie zur Herstellung bekannter Verbindungen hergestellt werden.

Verbindungen der Formel II mit l=1 können durch Kondensation einer Verbindung der Formel II, worin l für 0 steht, mit einer Verbindung der Formel

$$R^8\!-\!\underset{(b)}{\overset{\overset{\displaystyle R^{20}}{|}}{C}H}\!-\!COOH \qquad (IV)$$

worin $R^{20}$ dasselbe wie oben und $R^8$ eine geschützte Aminogruppe bedeutet, und die Konfiguration am C-Atom (b) wie oben ist, und anschliessendes Abspalten der in $R^8$ des Kondensationsprodukts anwesenden Schutzgruppe sowie, gewünschtenfalls, weiterer Schutzgruppen erhalten werden.

Verbindungen der Formel II mit l=2 können durch Kondensation einer Verbindung der Formel II mit l=0 mit einer Verbindung der allgemeinen Formel

$$R^8\!-\!\underset{(b)}{\overset{\overset{\displaystyle R^{20}}{|}}{C}H}\!-\!CO\!-\!NH\!-\!\underset{(b)}{\overset{\overset{\displaystyle R^{20}}{|}}{C}H}\!-\!COOH \qquad (V)$$

worin $R^8$ und $R^{20}$ dasselbe wie oben bedeuten und die Konfiguration am C-Atom (b) wie oben definiert ist, oder durch Kondensation einer Verbindung der Formel II mit l=1 mit einer Verbindung der Formel IV, und durch anschliessendes Abspalten der in $R^8$ des Kondensationsprodukts anwesenden Schutzgruppe sowie von weiteren gegebenenfalls anwesenden Schutzgruppen erhalten werden.

Verbindungen der Formel II mit l=3 können auf analoge Art hergestellt werden.

Die von $R^8$ dargestellte Aminoschutzgruppe kann von derselben Art wie $R^7$ sein. Zusätzlich kann $R^8$ auch die Phthalimidogruppe bedeuten.

Die vorerwähnte Kondensation mit einer Verbindung IV oder V kann auf analoge Weise durchgeführt werden, wie dies mit Bezug auf die Kondensation einer Verbindung II mit einer Verbindung III beschrieben worden ist.

Die Abspaltung der Schutzgruppe (n) aus dem erhaltenen Kondensationsprodukt kann auf analoge Art durchgeführt werden, wie dies mit Bezug auf die Abspaltung von Schutzgruppen aus dem Produkt der Kondensation von Verbindung II mit III beschrieben worden ist. Wenn $R^8$ im Kondensationsprodukt eine Phthalimidogruppe darstellt, kann dieselbe durch Hydrazinolyse in die Aminogruppe umgewandelt werden.

Die Verbindungen der Formeln III, IV und V sind entweder bekannt oder können nach an sich bekannten Methoden hergestellt werden.

Verbindungen der Formel II, worin l=2 oder 3 ist und wenigstens ein $R^{20}$ eine von den niedern Alkyl- bzw. Hydroxy-niederalkylgruppen, wie sie normalerweise in Proteinen vorkommen, sich unterscheidende Niederalkyl-bzw. Hydroxy-niederalkylgruppe oder eine solche Hydroxy-niederalkylgruppe mit geschütztem Hydroxy, bedeutet, sind neu und bilden ebenfalls Gegenstand der vorliegenden Erfindung.

Unter den genannten neuen Verbindungen der Formel II sind diejenigen bevorzugt, in denen sowohl $R^5$ wie $R^6$ Hydroxygruppen bedeuten, sowie diejenigen, in welchen $R^1$ Methyl darstellt, wie auch diejenigen, in welchen wenigstens ein $R^{20}$ eine Niederalkylgruppe, insbesondere Aethyl, n-Propyl oder n-Butyl bedeutet.

Beispiele von neuen Verbindungen der Formel II sind:

(1R)-1-(L-Norvalyl-L-alanylamino)-äthylphosphonsäure,
(1R)-1-(L-Alanyl-L-norvalylamino)-äthylphosphonsäure,
(1R)-1-(L-Norvalyl-L-norvalylamino)-äthylphosphonsäure,
(1R)-1-(Glycyl-L-norvalylamino)-äthylphosphonsäure,
(1R)-1-(L-Valyl-L-norvalylamino)-äthylphosphonsäure,
(1R)-1-(Glycyl-L-norvalyl-L-norvalylamino)-äthylphosphonsäure,
(1R)-1-(L-Valyl-L-valyl-L-norvalylamino)-äthylphosphonsäure, und
(L-Norvalyl-L-norvalylamino)-methylphosphonsäure.

Die erfindungsgemässen Peptidderivate sind antibakteriell gegen einen weiten Bereich von grampositiven und gram-negativen Bakterien wirksam, wie Escherichia coli, Serratia marcescens, Klebsiella aerogenes, Streptococcus faecalis und Haemophilus influenzae. Besonders gut sind sie gegen Streptococcus faecalis und Hameophilus influenzae, sowohl in vivo wie in vitro, wirksam. Sie unterscheiden sich von strukturell ähnlichen, z.B. aus den deutschen Offenlegungsschriften, Nos. 26 02 193, 27 21 760 und 27 21 761 bekannten Peptidderivaten im wesentlichen dadurch, dass die endständige freie Aminogruppe durch eine Alkyl-, Cycloalkyl-, Alkenyl- oder Arylgruppe substituiert ist oder dass eine an der endständigen Aminogruppe befindliche, in der Peptidchemie üblicherweise als Schutzfunktion verwendete Gruppe durch einen der vorstehend genannten Reste ersetzt ist. Überraschenderweise weisen die erfindungsgemäßen, Verbindungen gegenüber den vorbekannten Verbindungen vorteilhafte antibakterielle Wirkungen auf.

Die erfindungsgemässen Peptidderivate können als Arzneimittel verwendet werden, z.B. in Form pharmazeutischer Präparate, in welchen sie, zusammen mit einem pharmazeutisch verträglichen Trägermaterial, enthalten sind. Das Trägermaterial kann organisches oder anorganisches Material sein, das sich für enterale, z.B. orale, oder parenterale Verabreichung eignet. Beispiele solcher Trägermaterialien sind Wasser, Gelatine, Laktose, Stärke, Magnesiumstearat, Talk, vegetabile Oele, Gummi arabicum, Polyalkylenglykole etc. Die pharmazeutischen Präparate können in feste Form (z.B. Tabletten, Dragées, Suppositorien oder Kapseln) oder in flüssige Form gebracht werden (z.B. Lösungen, Suspensionen oder Emulsionen). Sie können den üblichen pharmazeutischen Operationen, wie Sterilisation, unterworfen werden und können Zusatzstoffe, wie Konservierungs-, Stabilisierungs-, Netz- oder Emulgiermittel, Salze zur Veränderung des osmotischen Druckes oder Puffer, enthalten. Bei Verwendung eines Puffers kann das pH des pharmazeutischen Präparates natürlich innerhalb des aus der pharmazeutischen Praxis wohlbekannten Bereiches variieren.

Die tägliche Dosis der an Erwachsene verabreichten Peptidderivate wird innerhalb weiter Grenzen variieren, und zwar in Abhängigkeit von Faktoren, wie dem gewählten Peptidderivat, der Verabreichungsart und der zu behandelnden Infektion. So kann z.B. die tägliche Dosis für orale Verabreichung bis zu 2000—4000 mg und die tägliche Dosis für parenterale Verabreichung bis zu 800—2000 mg betragen. Es versteht sich, dass die tägliche Dosis als Einzeldosis oder in mehreren Teilmengen verabreicht werden kann und dass die erwähnten Dosierungen sowohl nach unten wie nach oben variiert werden können, je nach individuellen Erfordernissen und in Anpassung an die besondern Umstände, wie sie vom behandelnden Arzt angetroffen werden.

Beispiel 1

1.1 Herstellung des Ausgangsmaterials

1.1.1 26,7 g (0,3 Mol) Sarcosin wurden in 150 ml (0,3 Mol) 2N Natriumhydroxid gelöst. Die erhaltene Lösung wurde bei 0°C gerührt, während 75 ml 4N Natriumhydroxid und 51,3 ml (ca. 360 mMol) Benzylchlorformiat alternierend und portionenweise im Verlaufe einer halben Stunde bei einer unterhalb 5°C gehaltenen Temperatur zugefügt wurden. Das Gemisch wurde eine weitere Stunde gerührt, wobei man die Temperatur auf Raumtemperatur ansteigen liess. Das Gemisch wurde dann mit Diäthyläther (2×300 ml) extrahiert. Die wässrige Schicht wurde abgetrennt, auf 0°C gekühlt und unter

Rühren mit ca. 25 ml konzentrierter Salzsäure auf Congorot angesäuert. Das Gemisch wurde eine Stunde gerührt, wobei man die Temperatur auf Raumtemperatur steigen liess. Das Gemisch wurde dann mit Diäthyläther (3×300 ml) extrahiert. Die vereinigten Extrakte wurden über Natriumsulfat getrocknet und eingedampft. Es ergaben sich ca. 64 g N-Benzyloxycarbonylsarcosin in Form eines Oels.

1.1.2 16 g (ca. 72 mMol) N-Benzyloxycarbonyl-sarcosin wurden in 150 ml Dimethoxyäthan gelöst. Die Lösung wurde auf 0°C gekühlt und sukzessive mit 8,28 g (72 mMol) N-Hydroxysuccinimid und 16,3 g (72 mMol) Dicyclohexylcarbodiimid behandelt. Das Gemisch wurde kann 16 Stunden bei 4°C gerührt. Das feste Nebenprodukt wurde wegfiltriert und das Filtrat zu einem Oel eingedampft. Dieses Oel, N-Benzyloxycarbonyl-sarcosin-N-hydroxysuccinimidester, wurde in Dimethylformamid gelöst und eine kleine Menge einer unlöslichen festen Substanz wegfiltriert. Das Filtrat wurde mit Dimethylformamid auf ein totales Volumen von 100 ml verdünnt und bei 4°C bis zum Gebrauch gelagert. Diese Lösung wird im folgenden als Vorratslösung bezeichnet.

1.2 Das Verfahren

1.2.1 4 g (15 mMol) (1R) - 1 - (L - Alanyl - L - analylamino) - äthylphosphonsäure wurden in einer Mischung von 15 ml Wasser und 4,2 ml (30 mMol) Triäthylamin gelöst. Die Lösung wurde bei 0°C gerührt, während 30 ml der vorgenannten Vorratslösung von N - Benzyloxycarbonyl - sarcosin - N - hydroxysuccinimidester zugefügt wurden. Das Gemisch wurde eine Stunde bei 0°C und dann sechzehn Stunden bei Raumtemperatur gerührt. Eine kleine Menge einer festen Substanz wurde abfiltriert und mit 10 ml Wasser/Dimethylformamid (1:2) gewaschen. Das Filtrat und die Waschwässer wurden vereinigt und unter Oelpumpenvakuum eingedampft. Der verbleibende Gummi wurde in 45 ml Wasser/Methanol (1:2) gelöst und durch eine Kolonne eines Kationenaustauscherharzes [B.D.H., Zerolit 225, SRC 13, $RSO_3H$; 100 g; frisch im Säurezyklus regeneriert und mit Wasser/Methanol (1:2) aufbereitet] geleitet. Die Elution mit Wasser/Methanol (1:2) ergab ein saures Eluat von ca. 400 ml, welches eingedampft wurde. Das verbleibende Oel wurde zwischen 200 ml Wasser und 100 ml Methylenchlorid verteilt. Die wässrige Schicht wurde mit 50 ml Methylenchlorid wieder extrahiert. Die Lösungsmittelextrakte wurden separat mit 100 ml Wasser nachgewaschen. Die vereinigten wässrigen Extrakte wurden mit 1N wässrigem Benzylamin auf pH 4,5 titriert und zu einer festen Substanz eingedampft. Umkristallisation dieser festen Substanz aus 100 ml Wasser mit Kühlung, gefolgt von Filtration und Waschen mit Wasser und dann mit Aceton ergaben 5,37 g des Monobenzylaminsalzes von (1R) - 1 - [(N - Benzyloxycarbonyl - sarcosyl - L - alanyl - L - alanyl)amino] - äthylphosphonsäure vom Schmelzpunkt 246—247°C (Zers.); $[\alpha]_D^{20}$=—79,1° (c=0,5% in Wasser); $[\alpha]_{365}^{20}$=—277° (c=0,5% in Wasser). Eindampfen des Filtrats und Umkristallisation des Rückstandes aus 40 ml Wasser ergaben weitere 1,687 g des gewünschten Monobenzylaminsalzes vom Schmelzpunkt 244—246°C (Zers.). Totalausbeute: 7,057 g (81%).

1.2.2 7 g (12 mMol) des Monobenzylaminsalzes von (1R) - 1 - [(N - Benzyloxycarbonyl - sarcosyl - L - alanyl - L - alanyl) - amino] - äthylphosphonsäure wurden in einer Mischung von 100 ml warmem Wasser und 20 ml 2N Ammoniumhydroxidlösung gelöst. Die Lösung wurde durch eine Kolonne des unter 1.2.1 genannten Kationenaustauscherharzes (100 g) geleitet. Die Elution erfolgte mit Wasser. Zum wässrigen Eluat wurden 0,2 g eines 10%igen Palladiumkohle-Katalysators, 6 Tropfen Eisessig und eine Menge an Aethanol gegeben, welche dem Volumen des Wässrigen Eluats gleich war. Das Gemisch wurde bei Raumtemperatur und Atmosphärendruck hydriert. Der Katalysator wurde abfiltriert. Die Lösungsmittel wurden durch Eindampfen entfernt. Die resultierende feste Substanz wurde aus einer Mischung von je 50 ml Wasser und Aethanol umkristallisiert. Es ergab sich eine erste Portion von 3,5 g (86%) eines Produkts vom Schmelzpunkt 300—310°C (Zers.). Eindampfen des Filtrats und Umkristallisation des Rückstandes aus einer Mischung von je 20 ml Wasser und Aethanol ergab eine zweite Portion von 0,29 g eines Produkts vom Schmelzpunkt 302—303°C (Zers.). Totalausbeute: 3,79 g (93%). Beide Portionen wurden vereinigt und aus einer Mischung von je 125 ml Wasser und Aethanol umkristallisiert. Man erhielt 3,385 g (1R) - 1 - (Sarcosyl - L - alanyl - L - alanylamino) - äthylphosphonsäure vom Schmelzpunkt 306—307°C (Zers.): $[\alpha]_D^{20}$=—118° (c=0,5% in Wasser). Eindampfen des Filtrats und Umkristallisation des Rückstandes aus einer Mischung aus 12 ml Wasser und 24 ml Aethanol ergaben weitere 0,16 g Produkt vom Schmelzpunkt 305—306°C (Zers.). Totalausbeute an umkristallisiertem Produkt: 3,545 g (87%).

Beispiel 2

2.1. Analog zu Beispiel 1.2.1 erhielt man aus 40 ml der Vorratslösung von N - Benzyloxycarbonyl - sarcosin - N - hydroxysuccinimid - ester (vergl. Beispiel 1.1.2) und 5,06 g (20 mMol) (1R) - 1 - (Glycyl - L - alanylamino) - äthylphosphonsäure, nach Titration mit Benzylamin, Eindampfen und Verreiben mit Aceton, 10,7 g (95%) Rohprodukt vom Schmelzpunkt ca. 195—205°C (Zers.). Dieses Produkt wurde aus einer Mischung von 80 ml Wasser, 200 ml Aethanol und 640 ml Diäthyläther umkristallisiert. Es ergaben sich 7,33 g (65%) des Monobenzylaminsalzes von (1R) - 1 - [(N - benzyloxycarbonyl - sarcosyl - glycyl - L - alanyl)amino] - äthylphosphonsäure vom Schmelzpunkt 222—224°C (Zers.); $[\alpha]_D^{20}$=—43.1° (c=0,5% in Wasser); $[\alpha]_{365}^{20}$=—155° (c=0,5% in Wasser). Durch Eindampfen des Filtrats der vorhergehenden Umkristallisation erhielt man weitere ca. 4,5 g des gewünschten Monobenzylaminsalzes vom Schmelzpunkt 220—224°C (Zers.).

2.2 Analog zu Beispiel 1.2.2, jedoch unter Verwendung nur von Wasser zum Lösen vor dem Ionenaustausch, erhielt man aus 4 g (7,5 mMol) des Monobenzylaminsalzes von (1R) - 1 - [(N - benzyloxycarbonyl - sarcosyl - glycyl - L - alanyl)amino] - äthylphosphonsäure nach Eindampfen mit n-Propanol und Umkristallisation aus einer Mischung von 15 ml Wasser und 30 ml Aethanol, 1,36 g (56%) (1R) - 1 - (sarcosyl - glycyl - L - alanylamino) - äthylphosphonsäure vom Schmelzpunkt 291—292°C (Zers.); $[\alpha]_D^{20}$=—77,9° (c=0,5% in Wasser); $[\alpha]_{365}^{20}$=—285° (c=0,5% in Wasser).

Beispiel 3

3.1 Analog zu Beispiel 1.2.1 erhielt aus 30 ml der Vorratslösung von N - Benzyloxycarbonyl - sarcosin - N - hydroxysuccinimid - ester (vergl. Beispiel 1.1.2) und 3,795 g (15 mMol) (L - Alanyl - L - alanylamino) - methylphosphonsäure, nach Verreiben mit Aceton, 8,39 g Rohprodukt vom Schmelzpunkt 228—230°C (Zers.). Umkristallisation aus 75 ml Wasser ergaben 6,2 g (73%) des Monobenzylaminsalzes von [(N - Benzyloxycarbonyl - sarcosyl - L - alanyl - L - alanyl)amino] - methylphosphonsäure vom Schmelzpunkt 235—236°C (Zers.); $[\alpha]_D^{20}$=—61,7° (c=1% in Wasser); $[\alpha]_{365}^{20}$=—215° (c=1% in Wasser).

3.2 5,65 g (10 mMol) des Monobenzylaminosalzes von [(N - Benzyloxycarbonyl - sarcosyl - L - alanyl - L - alanyl) - amino] - methylphosphonsäure wurden in einer Mischung von 75 ml Wasser, 75 ml Methanol und 2 ml konz. Salzsäure gelöst. 0,5 g 10%iger Palladiumkohle-Katalysator wurden zugetropft. Die Mischung wurde bei Raumtemperatur unter Normaldruck hydriert, der Katalysator dann abfiltriert und die Lösung eingedampft. Der feste Rückstand wurde mit 50 ml Methanol extrahiert, filtriert und dann mit Methanol (2×10 ml) gewaschen und getrocknet. Man erhielt so eine 1. Portion von 1,387 g Produkt vom Schmelzpunkt 245—253°C (Zers.). Die vereinigten Filtrate wurden mit 3 ml Propylenoxid behandelt, wobei sich eine weisse Festsubstanz niederschlug. Das Gemisch wurde bei Raumtemperatur zwei Stunden und dann im Kühlschrank über Nacht stehen gelassen. Die resultierende Festsubstanz wurde abfiltriert, mit Methanol gewaschen und getrocknet. Man erhielt so eine 2. Portion von 1,81 g Produkt vom Schmelzpunkt 284—285°C (Zers.). Die 1. Portion wurde in einer Mischung von je 50 ml Wasser und Methanol gelöst und die Lösung mit 13 ml Propylenoxid auf ein permanentes pH von ca. 5 eingestellt. Die Lösung wurde eingedampft, die resultierende Festsubstanz mit 50 ml Methanol verrieben und dann unter Methanol über Nacht stehen gelassen. Die Festsubstanz wurde anschliessend abfiltriert und getrocknet. Man erhielt 1,21 g Produkt vom Schmelzpunkt 285—286°C (Zers.). Dieses letztere Produkt wurde mit der oben erwähnten 2. Portion vereinigt und aus einer Mischung von je 35 ml Wasser und Aethanol umkristallisiert. Man erhielt 2,76 g (85% (Sarcosyl - L - alanyl - L - alanylamino) - methylphosphonsäure vom Schmelzpunkt 294—295°C (Zers.); $[\alpha]_D^{20}$=—78,7° (c=0,5% in Wasser); $[\alpha]_{365}^{20}$=—277° (c=0,5% in Wasser).

Beispiel 4

4.1 Herstellung des Ausgangsmaterials

5,88 g (30 mMol) (1R) - 1 - (L - Alanylamino) - äthylphosphonsäure wurden in 50 ml Wasser gelöst. Zur gerührten Lösung wurden 6,06 g (60 mMol) Triäthylamin und dann 50 ml Dimethylformamid gegeben. Die Lösung wurde auf 0°C gekühlt. Dann wurden 12,5 g (36 mMol) tert. Butoxycarbonyl - L - methionin - N - hydroxy - succinimidester in fester Form auf einmal zugefügt. Das Gemisch wurde zwei Stunden bei 0°C, dann über Nacht bei Raumtemperatur gerührt und anschliessend unter Oelpumpenvakuum zur Trockene eingedampft. Der Rückstand wurde in 150 ml Wasser aufgenommen und etwas Festsubstanz abfiltriert. Das Filtrat wurde mit 10 ml konz. Salzsäure angesäuert und die Lösung mit 100 ml Diäthyläther extrahiert. Der Extrakt wurde mit 75 ml Wasser gewaschen. Die wässrigen Schichten wurden vereinigt, 2 Tage bei Raumtemperatur stehen gelassen und dann zu einem Oel eingedampft. Dieses Oel wurde in 100 ml Aethanol gelöst, die Lösung gerührt und dann mit Propylenoxid (2×5 ml) auf ein permanentes pH von ca. 5 eingestellt. Die Lösung verwandelte sich in eine gelatinöse Masse, welche über Nacht bei Raumtemperatur stehen gelassen wurde. Filtration ergab eine Festsubstanz, welche mit 50 ml Aethanol gewaschen wurde. Man erhielt so 13,2 g Rohprodukt vom Schmelzpunkt 220—228°C (Zers.). Durch Umkristallisation aus einer Mischung von je 1 Liter Wasser und Aethanol erhielt man 8,17 g (1R) - 1 - (L - Methionyl - L - alanylamino) - äthylphosphonsäure vom Schmelzpunkt 250—252°C (Zers.); $[\alpha]_D^{20}$=—35,6° (c=0,5% in 1N NaOH-Lösung); $[\alpha]_{365}^{20}$=—136° (c=0,5% in 1N NaOH-Lösung). Eindampfen des Filtrats und Umkristallisation des Rückstandes aus einer Mischung von 250 ml Wasser und 500 ml Aethanol ergaben weitere 1,22 g Produkt vom Schmelzpunkt 250—252°C (Zers.). Totalausbeute: 9,39 g (95%).

4.2 Das Verfahren

4.2.1 Analog zu Beispiel 1.2.1 (wobei jedoch der nach Verdampfung des sauren Eluats aus dem Ionenaustausch-Schritt erhaltene Rückstand nicht zwischen Wasser und Methylenchlorid verteilt, sondern mit Diäthyläther extrahiert wurde) erhielt man aus 29 ml der Vorratslösung von N - Benzyloxycarbonyl - sarcosin - N - hydroxy - succinimidester (vergl. Beispiel 1.1.2) und 2,4 g (7,3 mMol) (1R) - 1 - (L - Methionyl - L - alanylamino) - äthylphosphonsäure, nach Verreiben mit 100 ml Aceton, 4,07 g Rohprodukt vom Schmelzpunkt 218—222°C (Zers.). Umkristallisation aus einer Mischung von je 400 ml Methanol und Aether ergab als gelatinösen Niederschlag 3,54 g (76%) des

Monobenzylamin-Salzes von (1R) - 1 - [(N - benzyloxycarbonyl - sarcosyl - L - methionyl - L - alanyl)amino] - äthylphosphonsäure vom Schmelzpunkt 231—234°C (Zers.); $[\alpha]_D^{20}$=—28,6° (c=0,6% in Essigsäure); $[\alpha]_{365}^{20}$=—111° (c=0,6% in Essigsäure).

4.2.2 3,76 g (5,9 mMol) des Monobenzylaminsalzes von (1R) - 1 - [(N - Benzyloxycarbonyl - sarcosyl - L - methionyl - L - alanyl)amino] - äthylphosphonsäure wurden mit einer Mischung von 6 ml Diäthylphosphit und 12 ml einer 45%igen Lösung von Bromwasserstoff in Eisessig bei Raumtemperatur fünf Stunden gerührt. Durch Zugabe von Diäthyläther wurde ein Gummi niedergeschlagen, welcher durch Dekantieren mit 75 ml Diäthyläther gewaschen wurde. Der verbleibende Gummi wurde unter Rühren in 75 ml Methanol aufgenommen. Propylenoxid (3x5 ml) wurde zugefügt. Es entstand eine gelatinöse Masse, welche schwierig zu rühren war. Das Gemisch wurde eine Stunde stehen gelassen und dann filtriert. Der Rückstand wurde mit Methanol und Diäthyläther gewaschen und dann getrocknet. Man erhielt so 2,05 g einer gummiartigen Festsubstanz. Umkristallisation aus einer Mischung von 80 ml Wasser und 400 ml Aethanol ergab, als gelatinösen Niederschlag, eine 1. Portion Produkt (0,53 g) vom Schmelzpunkt 262—265° (Zers.). Eindampfen der Mutterlaugen und Umkristallisation des Rückstandes aus einer Mischung von 30 ml Wasser und 180 ml Aethanol ergab dann eine 2. Portion Produkt (0,16 g) vom Schmelzpunkt 262—264°C (Zers.). Umkristallisation der 1. Portion aus einem Gemisch von 30 ml Wasser und 120 ml Aethanol ergab (1R) - 1 - (Sarcosyl - L - methionyl - L - alanylamino) - äthylphosphonsäure vom Schmelzpunkt 276—278°C (Zers.); $[\alpha]_D^{20}$=—56,0° (c=0,48% in frisch bereiteter 1N Natriumhydroxidlösung; $[\alpha]_{365}^{20}$=—217° (c=0,48% in frisch bereiteter 1N Natriumhydroxidlösung).

Beispiel 5

5.1 Herstellung des Ausgangsmaterials

5.1.1 9,1 g (20 mMol ($N^\alpha$,$N^{im}$ - bis(N - Benzyloxycarbonyl) - L - histidin wurden in 100 ml Dimethoxyäthan gelöst. Zur gerührten Lösung wurden 2,30 g (20 mMol) N-Hydroxysuccinimid zugegeben. Die Lösung wurde auf 0°C abgekühlt und mit 4,53 g (22 mMol) Dicyclohexylcarbodiimid versetzt. Das Gemisch wurde zwei Stunden bei 0°C gerührt und dann über Nacht stehengelassen. Der Niederschlag wurde abfiltriert und das Filtrat zu einem Gummi eingedampft. Dieser Gummi, $N^\alpha$,$N^{im}$ - bis(N - Benzyloxycarbonyl) - L - histidin - N - hydroxysuccinimid - ester, wurde in 40 ml Dimethylformamid gelöst und die erhaltene Lösung im folgenden Schritt verwendet.

5.1.2 3,14 g (16 mMol) (1R) - 1 - (L - Alanylamino) - äthylphosphonsäure wurden mit der vorerwähnten Lösung von $N^\alpha$,$N^{im}$ - bis(N - Benzyloxycarbonyl) - L - histidin - N - hydroxysuccinimid - ester analog zu der im Beispiel 1.2.1 beschriebenen Art umgesetzt. Nach Aufarbeitung in üblicher Art erhielt man (1R) - 1 - [(N - Benzyloxycarbonyl - L - histidyl - L - alanyl)amino] - äthylphosphonsäure vom Schmelzpunkt 256—258°C (Zers.).

5.1.3 3,27 g (7 mMol) (1R) - 1 - [(N - Benzyloxycarbonyl - L - histidyl - L - alanyl)amino] - äthylphosphonsäure wurden zu einem Pulver verrieben und dann bei Raumtemperatur sechs Stunden mit einer Mischung von 15 ml 45%igem Bromwasserstoff in Eisessig kräftig gerührt. Es wurden 75 ml Diäthyläther zugefügt, wobei ein Gummi niedergeschlagen wurde, der durch Dekantieren mit 75 ml Diäthyläther gewaschen wurde. Der verbleibende Gummi wurde in 15 ml Methanol gelöst. Zugabe von Propylenoxid (2x3 ml) ergab eine Festsubstanz. Die Mischung wurde über Nacht stehengelassen, die Festsubstanz abfiltriert und sukzessive mit Methanol und Diäthyläther gewaschen. Man erhielt so 2,47 g Rohprodukt von Schmelzpunkt 220—230°C (Zers.). Umkristallisation aus einer Mischung von 50 ml Wasser und 125 ml Aethanol ergab 2,02 g (87%) (1R) - 1 - (L - Histidyl - L - alanyl - amino) - äthylphosphonsäure vom Schmelzpunkt 242—245°C (Zers.); $[\alpha]_{365}^{20}$=—166° (c=0,5% in Wasser).

5.2 Das Verfahren

5.2.1 Analog zu Beispiel 1.2.1 wurden 27 ml der Vorratslösung von N - Benzyloxycarbonyl - sarcosin - N - hydroxysuccinimid - ester (vergl. Beispiel 1.1.2) mit 3 g (9 mMol) (1R) - 1 - (L - histidyl - L - alanylamino) - äthylphosphonsäure umgesetzt. Nach sechszehn Stunden Stehenlassen bei Raumtemperatur hatte sich die Mischung zu einer fast festen Masse gesetzt. Es wurden 200 ml Wasser zugefügt und die resultierende Mischung wurde dann gerührt und filtriert. Das Filtrat wurde unter Oelpumpenvakuum (0,1 mm Hg) eingedampft, die erhaltene Festsubstanz mit 150 ml Aceton verrieben, eine halbe Stunde bei Raumtemperatur stehengelassen und dann filtriert. Man erhielt 4,6 g (95%) Produkt vom Schmelzpunkt 238—240°C (Zers.). Umkristallisation einer Probe dieses Produkts aus 10 ml heissem Wasser ergab (1R) - 1 - [(N - Benzyloxycarbonyl - sarcosyl - L - histidyl - L - alanyl)amino] - äthylphosphonsäure vom Schmelzpunkt 243—245°C (Zers.); $[\alpha]_D^{20}$=—44,1° (c=0,46% in frisch bereitetem 0,1N NaOH); $[\alpha]_{365}^{20}$=—182° (c=0,46% in frisch bereitetem 0,1N NaOH).

5.2.2 Auf analoge Weise wie unter 5.1.3 beschrieben, erhielt man aus 4 g (7,4 mMol) (1R) - 1 - [(N - Benzyloxycarbonyl - sarcosyl - L - histidyl - L - alanyl)amino] - äthylphosphonsäure 2,82 g Rohprodukt vom Schmelzpunkt 276—278°C (Zers.). Umkristallisation aus einer Mischung von 30 ml Wasser und 60 ml Aethanol ergab 2,47 g (69%) des Monohydrochlorids von (1R) - 1 - (Sarcosyl - L - histidyl - L - alanylamino) - äthylphosphonsäure vom Schmelzpunkt 280—281°C (Zers.); $[\alpha]_D^{20}$=—48,2° (c=0,5% in Wasser); $[\alpha]_{365}^{20}$=—179° (c=0,5% in Wasser).

Beispiel 6

6.1 Herstellung des Ausgangsmaterials

6.1.1 Analog zu Beispiel 1.1.2 wurden 6,2 g (14,7 mMol) $N^{\alpha}$,$N^{im}$ - bis(N - Benzyloxycarbonyl) - L - histidin in den N-Hydroxysuccinimid-ester übergeführt. Der resultierende Aktivester (ca. 14,7 mMol) wurde in 25 ml Dimethylformamid gelöst und die resultierende Lösung rasch und tropfenweise bei 0°C zu einer gerührten Lösung von 1,5 g (12 mMol) (1R) - 1 - Aminoäthylphosphonsäure in je 25 ml Wasser und Dimethylformamid und 2,4 g Triäthylamid zugefügt. Das Gemisch wurde zwei Stunden bei 0°C und dann über Nacht bei Raumtemperatur gerührt. Eine geringe Menge einer Festsubstanz wurde abfiltriert, das Filtrat unter Oelpumpenvakuum eingedampft und der Rückstand nochmals aus Wasser und dann aus n-Propanol eingedampft. Es wurden 11,6 g Rückstand erhalten. Dieser Rückstand wurde mit 100 ml Aceton verrieben, der weisse kristalline Niederschlag abfiltriert und mit 50 ml Aceton gewaschen. Es wurden so 4,02 g (85%) von im wesentlichen reinem (1R) - 1 - [($N^{\alpha}$ - Benzyloxycarbonyl - L - histidyl) - amino] - äthylphosphonsäure erhalten; Schmelzpunkt 236—239°C (Zers.); $[\alpha]_D^{20}$=—18,2° (c=0,49% in Essigsäure). Umkristallisation einer Probe aus Wasser, Aethanol und Diäthyläther ergab diese Substanz mit einem Schmelzpunkt von 244—246°C (Zers.); $[\alpha]_D^{20}$=—18,6° (c=0,37% in Essigsäure).

6.1.2 7,2 g (18,2 mMol) (1R) - 1 - [($N^{\alpha}$ - Benzyloxycarbonyl - L - histidyl)amino] - äthylphosphonsäure wurden in einer Mischung von je 50 ml Eisessig und Wasser gelöst. Nach Zugabe von 1 g 10%igem Palladiumkohle-Katalysator wurde das Gemisch über Nacht bei Raumtemperatur und Atmosphärendruck hydriert. Der Katalysator wurde abfiltriert, das Filtrat eingedampft und der Rückstand noch dreimal aus Wasser und dann aus n-Propanol eingedampft. Die resultierende Festsubstanz wurde mit 60 ml Methanol verrieben und filtriert. Man erhielt 4,17 g Rohprodukt vom Schmelzpunkt 180°C. Umkristallisation aus einer Mischung von 70 ml Wasser und 105 ml Aethanol ergab 3,75 g (79%) von leicht hygroskopischem (1R) - 1 - (L - Histidylamino) - äthylphosphonsäure vom Schmelzpunkt 185°C (Zers.); $[\alpha]_D^{20}$=—120° (c=0,36% in Wasser).

6.1.3 Analog zu Beispiel 1.2.1 wurden 7,2 g (22,5 mMol) N - Benzyloxycarbonyl - L - alanin - N - hydroxysuccinimid - ester mit 3,93 g (15 mMol) (1R) - 1 - (L - Histidylamino) - äthylphosphonsäure umgesetzt. Nach Filtration wurde die Mischung zu einem Oel eingedampft. Behandlung dieses Oels mit 150 ml Aceton ergab 6,28 g Rohprodukt vom Schmelzpunkt ca. 150°C. Umkristallisation einer Probe dieses Rohprodukts aus Wasser, Aethanol und Diäthyläther ergab (1R) - 1 - [(N - Benzyloxycarbonyl - L - alanyl - L - histidyl) - amino] - äthylphosphonsäure vom Schmelzpunkt 253—255°C (Zers.); $[\alpha]_D^{20}$=—44.1° (c=0,5% in Wasser); $[\alpha]_{365}^{20}$=—152° (c=0,5% in Wasser).

6.1.4 Analog zu Beispiel 5.1.3 erhielt man aus 5,87 g (12,6 mMol) des Produkts von 6.1.3 4,59 g Rohprodukt vom Schmelzpunkt 197°C (Zers.). Umkristallisation aus einer Mischung von Wasser und 100 ml Aethanol ergaben 1,68 g (40%) (1R) - 1 - (L - Alanyl - L - histidylamino) - äthylphosphonsäure vom Schmelzpunkt 202°C (Zers.); $[\alpha]_D^{20}$=—37,3° (c=0,5% in Wasser); $[\alpha]_{365}^{20}$=—148° (c=0,5% in Wasser).

6.2 Das Verfahren

6.2.1 Analog zu Beispiel 1.2.1 erhielt man aus 18 ml der Vorratslösung von N - Benzyloxycarbonyl - sarcosin - N - hydroxysuccinimid - ester (vergl. Beispiel 1.1.2) und 2 g (6 mMol) (1R) - 1 - (L - Alanyl - L - histidylamino) - äthylphosphonsäure, nach Verreiben mit Aceton, 2,57 g von im wesentlichen reiner (1R) - 1 - [(N - Benzyloxycarbonyl - sarcosyl - L - alanyl - L - histidyl)amino] - äthylphosphonsäure vom Schmelzpunkt 256°C (Zers.).

6.2.2 Analog zu Beispiel 5.1.3 erhielt man aus 2,5 g (4,6 mMol) (1R) - 1 - [(N - Benzyloxycarbonyl - sarcosyl - L - alanyl - L - histidyl)amino] - äthylphosphonsäure 2,25 g Rohprodukt vom Schmelzpunkt 187°C. Umkristallisation aus einer Mischung von 25 ml Wasser (mit Filtration) und 150 ml Aethanol ergaben 1,20 g (1R) - 1 - (Sarcosyl - L - alanyl - L - histidylamino) - äthylphosphonsäure vom Schmelzpunkt 250°C (Zers.); $[\alpha]_D^{20}$=—58,6° (c=0,5% in Wasser); $[\alpha]_{365}^{20}$=—214° (c=0,5% in Wasser).

Beispiel 7

7.1 Herstellung des Ausgangsmaterials

7.1.1.1 10,6 g (30 mMol) N - Benzyloxycarbonyl - L - nitro - arginin suspendiert in 500 ml Petroläther wurden mit 3,03 (30 mMol) Triäthylamin gerührt. Die Mischung wurde auf —5°C gekühlt. 4,11 g (30 mMol) Isobutylchlorformiat wurden tropfenweise zugegeben. Die Mischung wurde dann noch eine halbe Stunde bei —5°C gehalten. Das resultierende gemischte Anhydrid wurde rasch, tropfenweise und unter Rühren bei —5°C mit einer Lösung von 3,75 g (30 mMol) (1R) - 1 - Aminoäthylphosphonsäure in 50 ml Wasser (enthaltend 6,06 g (60 mMol) Triäthylamin) behandelt. Das Gemisch wurde weitere zwei Stunden bei —5°C gerührt und dann über Nacht noch bei Raumtemperatur. Dann wurden 150 ml Wasser zugefügt und die Phasen getrennt. Die wässrige Phase wurde zur Trockene eingedampft und der Rückstand in einem Gemisch von je 50 ml Wasser und Methanol aufgenommen. Die Lösung wurde durch eine Kolonne eines Kationenaustauscherharzes (150 g), wie unter 1.2.1 beschrieben, geleitet. Die Kolonne wurde mit Wasser/Methanol (1:1) eluiert, das saure Eluat zur Trockene verdampft und der Rückstand nochmals aus Wasser eingedampft. Der gummiartige Rückstand wurde zwischen 150 ml Wasser und 100 ml Aethylacetat verteilt und der Aethylacetatextrakt mit 100 ml Wasser gewaschen. Weitere Extraktion mit Wasser (2×100 ml) ergab einen 2. vereinigten wässrigen Extrakt. Die wässrigen Extrakte wurden eingedampft. Der Rückstand wurde in Wasser/Methanol (1:1) gelöst und die Lösung mit 4N wässrigen Benzylamin auf pH 4,5 eingestellt. Eindampfen und Kristallisation des Rückstandes aus Methanol/Diäthyläther ergaben 4,12 g des Monobenzylaminsalzes von (1R) - 1 - [(N - Benzyloxycarbonyl - L - nitroarginyl) - amino] - äthylphosphonsäure vom Schmelzpunkt 214—217°C (Zers.); $[\alpha]_D^{20}$=—16,4° (c=0,52% in Methanol).

7.1.1.2 26,5 g (75 mMol) N - Benzyloxycarbonyl - L - nitroarginin wurden in 200 ml Dimethylformamid gelöst. Zur gerührten Lösung wurden 8,6 g (75 mMol) N-Hydroxysuccinimid gegeben. Die resultierende Lösung wurde auf 0°C gekühlt und mit 17 g (82,5 mMol) Dicyclohexylcarbodiimid behandelt. Die erhaltene Mischung wurde zwei Stunden bei 0°C gerührt und dann über Nacht bei Raumtemperatur. Nach Kühlung auf 0°C wurde ein festes Nebenprodukt abfiltriert und das Filtrat unter Oelpumpenvakuum eingedampft. Der so als schwach gelbes Oel erhaltene N - Benzyloxycarbonyl - L - nitroarginin - N - hydroxysuccinimid - ester wurde in 60 ml Dimethylformamid gelöst und bei 0°C rasch mit einer Lösung von 7,8 g (62,5 mMol) (1R) - 1 - Aminoäthylphosphonsäure in 60 ml Wasser, 12,6 g Triäthylamin und 60 ml Dimethylformamid versetzt. Das Gemisch wurde bei 0°C mehrere Stunden gerührt und dann über Nacht bei Raumtemperatur. Etwas Festsubstanz wurde abfiltriert und das Filtrat unter Oelpumpenvakuum bei einer Badtemperatur von 35°C zur Trockene eingedampft. Das verbleibende Oel wurde mit 100 ml Methanol trituriert. Die weisse Festsubstanz wurde abfiltriert. Das Filtrat wurde mit 50 ml Wasser verdünnt und die Lösung durch eine Kolonne eines Kationenaustauscherharzes wie sub 1.2.1 beschrieben (300 g) geleitet. Die Eluierung der Kolonne mit Methanol/Wasser (2:1) ergab ein saures Eluat von 750 ml, welches eingedampft wurde. Der Rückstand wurde zwischen 300 ml Wasser und 150 ml Aethylacetat verteilt und die organische Schicht weiter mit 100 ml Wasser extrahiert. Sämtliche wässrigen Extrakte wurden dann mit 100 ml Aethylacetat gewaschen. Die vereinigten Aethylacetatextrakte wurden mit Wasser (2×150 ml) gewaschen. Die vereinigten wässrigen Extrakte wurden mit Benzylamin behandelt, wie im vorangehenden Absatz beschrieben. Man erhielt 11,54 g des Monobenzylaminsalzes von (1R) - [(N-Benzyloxycarbonyl - L - nitroarginyl)amino] - äthylphosphonsäure vom Schmelzpunkt 209—212°C (Zers.).

7.1.2 4 g (7 mMol) des Monobenzylaminsalzes von (1R) - 1 - [(N - Benzyloxycarbonyl - L - nitroarginyl)amino] - äthylphosphonsäure wurden zu 10 ml einer 45%igen Lösung von Bromwasserstoff in Eisessig gegeben. Das Gemisch wurde sechs Stunden bei Raumtemperatur gerührt. Dann wurden 75 ml Diäthyläther zugegeben, wobei ein Gummi ausfiel. Das Ueberstehende wurde durch Dekantieren entfernt und der Gummi mit weiteren 75 ml Diäthyläther gewaschen, dann in 40 ml Methanol gelöst und die gerührte Lösung mit 5 ml Propylenoxid behandelt. Nach fünf Minuten entstand ein weisser Niederschlag. Das Gemisch wurde eine Stunde bei Raumtemperatur gerührt bis das Ueberstehende ein pH von 3 aufwies. Nach Zugabe von weiteren 3 ml Propylenoxid wurde das Gemisch über Nacht stehengelassen. Die ausgeschiedene Festsubstanz wurde abfiltriert, mit Methanol und dann mit Diäthyläther gewaschen. Man erhielt so 2,19 g (95%) (1R) - 1 - (L - Nitroarginylamino) - äthylphosphonsäure vom Schmelzpunkt 240—243°C (Zers.); $[\alpha]_D^{20}$=—13,2° (c=0,51% in Wasser).

7.1.3 11,5 g (36 mMol) N - Benzyloxycarbonyl - L - alanin - N - hydroxysuccinimid - ester wurden umgesetzt mit 8 g (24,5 mMol) (1R) - 1 - (L - Nitroarginylamino) - äthylphosphonsäure in Analogie zum ersten Absatz dieses Beispiels. Die Aufarbeitung ergab 13,92 g des Monobenzylaminsalzes vom Schmelzpunkt 210—215°C (Zers.). Umkristallisation einer Probe aus einer Mischung von Wasser, Aethanol und Diäthyläther ergab das Monobenzylaminsalz von (1R) - 1 - [(N - Benzyloxycarbonyl - L - alanyl - L - nitroarginyl) - amino] - äthylphosphonsäure vom Schmelzpunkt 228—230°C (Zers.); $[\alpha]_D^{20}$=—31,5° (c=0,5% in Essigsäure); $[\alpha]_{365}^{20}$=—117° (c=0,5% in Essigsäure).

7.1.4 In Analogie zum obigen Absatz 7.1.2 erhielt man aus 13,4 g (21 mMol) des Monobenzylaminsalzes von (1R) - 1 - [(N - Benzyloxycarbonyl - L - alanyl - L - nitroarginyl) - amino] - äthylphosphonsäure, nach Behandlung mit Propylenoxid, 11,3 g Rohprodukt vom Schmelzpunkt 200—210°C (Zers.). Umkristallisation aus einer Mischung von Wasser und Aethanol ergab 7,04 g (1R) - 1 - (L - Alanyl - L - nitroarginylamino) - äthylphosphonsäure vom Schmelzpunkt 263—265°C (Zers.); $[\alpha]_D^{20}$=—22,9° (c=0,5% in Wasser); $[\alpha]_{365}^{20}$=—71,9° (c=0,5% in Wasser).

7.2 Das Verfahren

7.2.1 In Analogie zu Absatz 7.1.1.2 erhielt man aus 45 ml der Vorratslösung von N - Benzyloxycarbonyl - sarcosin - N - hydroxysuccinimid - ester (vergl. Beispiel 1.1.2) und 5,96 g (15 mMol) (1R) - 1 - (L - Alanyl - L - nitroarginylamino) - äthylphosphonsäure 11 g rohes Monobenzylaminsalz von (1R) - 1 - [(N - Benzyloxycarbonyl - sarcosyl - L - alanyl - L - nitroarginyl) - amino] - äthylphosphonsäure vom Schmelzpunkt 230°C (Zers.). Umkristallisation einer Probe aus einer Mischung von Wasser, Aethanol und Diäthyläther ergab das Monobenzylaminsalz von (1R) - 1 - [(N - Benzyloxycarbonyl - sarcosyl - L - alanyl - L - nitroarginyl)amino] - äthylphosphonsäure vom Schmelzpunkt 236—239°C (Zers.): $[\alpha]_D^{20}$=—38,2° (c=0,5% in Essigsäure); $[\alpha]_{365}^{20}$=—141° (c=0,5% in Essigsäure).

7.2.2 Analog zu Absatz 7.1.2 erhielt man aus 10,5 g (15 mMol) des Monobenzylaminsalzes von

(1R) - 1 - [(N - Benzyloxycarbonyl - sarcosyl - L - alanyl - L - nitroarginyl)amino] - äthylphosphonsäure 6,72 g Rohprodukt vom Schmelzpunkt 225—235°C (Zers.). Umkristallisation aus einer Mischung von 50 ml Wasser und 150 ml Aethanol ergab 5,25 g (1R) - 1 - (Sacrosyl - L - alanyl - L - nitroarginylamino) - äthylphosphonsäure vom Schmelzpunkt 253—255°C (Zers.); $[\alpha]_D^{20}$=—59,9° (c=0,55% in Wasser); $[\alpha]_{365}^{20}$=—195° (c=0,55% in Wasser).

7.2.3 3,92 g (8,4 mMol) (1R) - 1 - (Sarcosyl - L - alanyl - L - nitroarginylamino) - äthylphosphonsäure wurden in 120 ml Wasser gelöst. 0,8 g 10%iger Palladiumkohle-Katalysator wurden zugefügt. Die Mischung wurde bei Raumtemperatur und Atmosphärendruck hydriert bis die Aufnahme des Wasserstoffs aufhörte. Der Katalysator wurde abfiltriert und das Lösungsmittel durch Filtration entfernt. Der gummiartige Rückstand wurde mit 150 ml Methanol trituriert und dann bei Raumtemperatur stehengelassen. Die ausgeschiedene Festsubstanz wurde abfiltriert, mit Methanol und dann mit Diäthyläther gewaschen. Man erhielt 3,05 g Rohprodukt vom Schmelzpunkt ca. 200°C (Zers.). Umkristallisation aus einer Mischung von 40 ml warmem Wasser und 140 ml Aethanol ergab 2,44 g (1R) - 1 - (Sarcosyl - L - alanyl - L - arginylamino) - äthylphosphonsäure vom Schmelzpunkt ca. 205°C (Zers.); $[\alpha]_D^{20}$=—78,8° (c=0,5% in Wasser); $[\alpha]_{365}^{20}$=—293° (c=0,5% in Wasser).

Beispiel 8

8.1 Herstellung des Ausgangsmaterials

8.1.1 Analog zu Beispiel 1.2.1 erhielt man aus N - Benzyloxycarbonyl - L - serin - N - hydroxysuccinimid - ester (hergestellt in situ als Gummi aus N - Benzyloxycarbonyl - L - serin, N-Hydroxysuccinimid und Dicyclohexylcarbodiimid mit Dimethoxymethan als Lösungsmittel) und (1R) - 1 - Aminoäthylphosphonsäure das Monobenzylaminsalz von (1R) - 1 - [(N - Benzyloxycarbonyl - L - seryl) - amino] - äthylphosphonsäure vom Schmelzpunkt 207—210°C (Zers.); $[\alpha]_D^{20}$=—24,0° (c=0,5% in Essigsäure).

8.1.2 Analog zu Beispiel 3.2 erhielt man aus dem Monobenzylaminsalz von (1R) - 1 - [(N - Benzyloxycarbonyl - L - seryl)amino] - äthylphosphonsäure die (1R) - 1 - (L - Serylamino) - äthylphosphonsäure vom Schmelzpunkt 250—251°C (Zers.); $[\alpha]_D^{20}$=—41,7° (c=0,5% in Wasser).

8.1.3 Analog zu Beispiel 1.2.1 erhielt man aus N - Benzyloxycarbonyl - L - alanin und N - Hydroxysuccinimid - ester und (1R) - 1 - (L - Serylamino) - äthylphosphonsäure das Monobenzylaminsalz von (1R) - 1 - [(N - Benzyloxycarbonyl - L - alanyl - L - seryl)amino] - äthylphosphonsäure vom Schmelzpunkt 226—228°C (Zers.); $[\alpha]_D^{20}$=—47,0° (c=0,5% in Wasser).

8.1.4 Analog zu Beispiel 3.2 erhielt man aus dem Monobenzylaminsalz von (1R) - 1 - [(N - Benzyloxycarbonyl - L - alanyl - L - seryl)amino] - äthylphosphonsäure die (1R) - 1 - (L - Alanyl - L - serylamino) - äthylphosphonsäure vom Schmelzpunkt 234—235°C (Zers.).

8.2 Das Verfahren

8.2.1 Analog zu Beispil 1.2.1 erhielt man aus der Vorratslösung von N - Benzyloxycarbonyl - sarcosin - N - hydroxysuccinimid - ester und (1R) - 1 - (L - Alanyl - L - serylamino) - äthylphosphonsäure das Monobenzylaminsalz von (1R) - 1 - [(N - Benzyloxycarbonyl - sarcosyl - L - alanyl - L - seryl)amino] - äthylphosphonsäure vom Schmelzpunkt 219—221°C (Zers.); $[\alpha]_D^{20}$=—60,5° (c=0,5% in Wasser).

8.2.2 Analog zu Beispiel 3.2 erhielt man aus dem Monobenzylaminsalz von (1R) - 1 - [(N - Benzyloxycarbonyl - sarcosyl - L - alanyl - L - seryl)amino] - äthylphosphonsäure die (1R) - 1 - (Sarcosyl - L - alanyl - L - serylamino) - äthylphosphonsäure vom Schmelzpunkt 259—260°C (Zers.); $[\alpha]_D^{20}$=—92,3° (c=0,5% in Wasser).

Beispiel 9

9.1 Herstellung des Ausgangsmaterials

9.1.1 Analog zu Beispiel 1.2.1 erhielt man aus N - Benzyloxycarbonyl - L - alanin - N - hydroxysuccinimid - ester und (1R) - 1 - (L - Norvalylamino) - äthylphosphonsäure das Monobenzylaminsalz von (1R) - 1 - [(N - Benzyloxycarbonyl - L - alanyl - L - norvalyl)amino] - äthylphosphonsäure vom Schmelzpunkt 240—243°C (Zers.). Eine Probe wurde aus Warmem Wasser umkristallisiert, wobei man 0,11 g eines Produkts vom Schmelzpunkt 234—237°C (Zers.) erhielt; $[\alpha]_D^{20}$=—39,1°; $[\alpha]_{365}^{20}$=—137° (c=0,49% in Essigsäure).

9.1.2 Analog zu Beispiel 5.1.3 erhielt man aus dem Monobenzylaminsalz von (1R) - 1 - [(N - Benzyloxycarbonyl - L - alanyl - L - norvalyl)amino] - äthylphosphonsäure die (1R) - 1 - (L - Alanyl - L - norvalyl - amino) - äthylphosphonsäure vom Schmelzpunkt 268—270°C; $[\alpha]_D^{20}$=—50,4°; $[\alpha]_{365}^{20}$=—201° (c=0,46%, in frisch bereiteter 1N Natriumhydroxidlösung).

9.2 Das Verfahren

9.2.1 Analog zu Beispiel 1.2.1 erhielt man aus der Vorratslösung von N - Benzyloxycarbonyl - sarcosin - N - hydroxysuccinimid - ester in Dimethylformamid und (1R) - 1 - (L - Alanyl - L - norvalylamino) - äthylphosphonsäure das Monobenzylaminsalz von (1R) - 1 - [(N - Benzyloxycarbonyl - sarcosyl - L - alanyl - L - norvalyl)amino] - äthylphosphonsäure vom Schmelzpunkt 257—259°C (Zers.); $[\alpha]_D^{20}$=—43,7°; $[\alpha]_{365}^{20}$=—164° (c=0,49% in Essigsäure).

9.2.2 Analog zu Beispiel 5.1.3 erhielt man aus dem Monobenzylaminsalz von (1R) - 1 - [(N - Benzyloxycarbonyl - sarcosyl - L - alanyl - L - norvalyl)amino] - äthylphosphonsäure die (1R) - 1 - (Sarcosyl - L - alanyl - L - norvalylamino) - äthylphosphonsäure vom Schmelzpunkt 291—292°C (Zers.); $[\alpha]_D^{20}$=—110°; $[\alpha]_{365}^{20}$=—391° (c=0,26% in Wasser).

Beispiel 10

10.1 Herstellung des Ausgangsmaterials

10.1.1 Analog zu Beispiel 1.2.1 erhielt man aus N - Benzyloxycarbonyl - L - serin - N - hydroxysuccinimid - ester (hergestellt in situ analog zu Beispiel 8.1.1) und (1R) - 1 - (L - Alanyl - amino) - äthylphosphonsäure das Monobenzylaminsalz von (1R) - 1 - [(N - Benzyloxycarbonyl - L - seryl - L - alanyl)amino] - äthylphosphonsäure vom Schmelzpunkt 200—205°C (Zers.); $[\alpha]_D^{20}$=—53,7°; $[\alpha]_{365}^{20}$=—189° (c=0,3% in Wasser).

10.1.2 Analog zu Beispiel 3.2 erhielt aus dem Monobenzylaminsalz von (1R) - 1 - [(N - Benzyloxycarbonyl - L - seryl - L - alanyl)amino] - äthylphosphonsäure die (1R) - 1 - (L - Seryl - L - alanylamino) - äthylphosphonsäure vom Schmelzpunkt 256—257°C (Zers.); $[\alpha]_D^{20}$=—61,4°; $[\alpha]_{365}^{20}$=—222° (c=0,5% in Wasser).

10.2 Das Verfahren

10.2.1 Analog zu Beispiel 1.2.1 erhielt man aus der Vorratslösung von N - Benzyloxycarbonyl - sarcosin - N - hydroxysuccinimid - ester und (1R) - 1 - (L - Seryl - L - alanylamino) - äthylphosphonsäure das hygroskopische Monobenzylaminsalz von (1R) - 1 - [(N - Benzyloxycarbonyl - sarcosyl - L - seryl - L - alanyl)amino] - äthylphosphonsäure vom Schmelzpunkt 204—205°C (Zers.); $[\alpha]_D^{20}$=—55,9°; $[\alpha]_{365}^{20}$=—201° (c=0,5% in Wasser).

10.2.2 Analog zu Beispiel 3.2 erhielt man aus dem Monobenzylaminsalz von (1R) - 1 - [(N - Benzyloxycarbonyl - sarcosyl - L - seryl - L - alanyl)amino] - äthylphosphonsäure die (1R) - 1 - (Sarcosyl - L - seryl - L - alanyl - amino] - äthylphosphonsäure vom Schmelzpunkt 249—250°C (Zers.); $[\alpha]_D^{20}$=—85,2°; $[\alpha]_{365}^{20}$=—301° (c=0,5% in Wasser).

Beispiel 11

11.1 Herstellung des Ausgangsmaterials

11.1.1 Analog zu Beispiel 7.1.1.1, jedoch unter Verwendung von N-Aethylmorpholin anstelle von Triäthylamin, erhielt man aus N - Benzyloxycarbonyl - L - nitroarginin und (L - alanylamino) - äthylphosphonsäure das Monobenzylaminsalz von (1R) - 1 - [(N - Benzyloxycarbonyl - L - nitroarginyl - L - alanyl) - amino] - äthylphosphonsäure vom Schmelzpunkt 193—203°C (Zers.).

11.1.2 Analog zu Beispiel 5.1.3 erhielt man aus dem Monobenzylaminsalz von (1R) - 1 - [(N - Benzyloxycarbonyl - L - nitroarginyl - L - alanyl)amino] - äthylphosphonsäure die (1R) - 1 - (L - Nitroarginyl - L - alanyl - amino) - äthylphosphonsäure vom Schmelzpunkt 224—226°C (Zers.); $[\alpha]_D^{20}$=—26,0°; $[\alpha]_{365}^{20}$=—75,6° (c=0,43% in Wasser).

11.2 Das Verfahren

11.2.1 Analog zu Beispiel 7.2.1 erhielt man aus der Vorratslösung von N - Benzyloxycarbonyl - sarcosin - N - hydroxysuccinimid - ester und (1R) - 1 - (L - Nitroarginyl - L - alanylamino) - äthylphosphonsäure das Monobenzylaminsalz von (1R) - 1 - [(N - Benzyloxycarbonyl - sarcosyl - L - nitroarginyl - L - alanyl)amino] - äthylphosphonsäure vom Schmelzpunkt 212—215°C (Zers.); Umkristallisation einer Probe aus Wasser/Aethanol/Aether ergab ein Produkt vom Schmelzpunkt 219—221°C (Zers.); $[\alpha]_D^{20}$=—35,6°; $[\alpha]_{365}^{20}$=—121° (c=0,55% in Wasser).

11.2.2 Analog zu Beispiel 7.1.2 erhielt man aus dem Monobenzylaminsalz von (1R) - 1 - [(N - Benzyloxycarbonyl - sarcosyl - L - nitroarginyl - L - alanyl)amino] - äthylphosphonsäure die (1R) - 1 - (Sarcosyl - L - nitroarginyl - L - alanylamino) - äthylphosphonsäure vom Schmelzpunkt 281—283°C (Zers.); $[\alpha]_D^{20}$=—57,7°; $[\alpha]_{365}^{20}$=—193°.

11.2.3 Analog zu Beispiel 7.2.3 erhielt man aus (1R) - 1 - (Sarcosyl - L - nitroarginyl - L - alanylamino) - äthylphosphonsäure die (1R) - 1 - (Sarcosyl - L - arginyl - L - alanylamino) - äthylphosphonsäure vom Schmelzpunkt ca. 183°C (Zers.); $[\alpha]_D^{20}$=—59,8°; $[\alpha]_{365}^{20}$=—231° (c=0,51% in Wasser).

Beispiel 12

12.1 Herstellung des Ausgangsmaterials

12.1.1 Analog zu Beispiel 9.1.1 erhielt man aus N - Benzyloxycarbonyl - L - norvalin - N - hydroxysuccinimid - ester und (1R) - 1 - (L - Alanylamino) - äthylphosphonsäure das Monobenzylaminsalz von (1R) - 1 - [(N - Benzyloxycarbonyl - L - norvalyl - L - alanyl)amino] - äthylphosphonsäure vom Schmelzpunkt 225—229°C (Zers.). Umkristallisation einer Probe aus warmem Wasser/Aethanol/Aether ergab 0,35 g Produkt vom Schmelzpunkt 230—232°C (Zers.); $[\alpha]_D^{20}$=—40,3°; $[\alpha]_{365}^{20}$=—148° (c=0,51% in Essigsäure).

12.1.2 Analog zu Beispiel 5.1.3 erhielt man aus dem Monobenzylaminsalz von (1R) - 1 - [(N - Benzyloxycarbonyl - L - norvalyl - L - alanyl)amino] - äthylphosphonsäure die (1R) - 1 - (L -

Norvalyl - L - alanylamino) - äthylphosphonsäure vom Schmelzpunkt 260—261°C (Zers.); $[\alpha]_D^{20}$=—46,5°; $[\alpha]_{365}^{20}$=—168° (c=0,51% in Wasser).

12.2 Das Verfahren

12.2.1 Analog zu Beispiel 1.2.1 erhielt man aus der Vorratslösung von N - Benzyloxycarbonyl - sarcosin - N - hydroxysuccinimid - ester und (1R) - 1 - (L - Norvalyl - L - alanyl - amino) - äthylphosphonsäure das Monobenzylaminsalz von (1R) - 1 - [(N - Benzyloxycarbonyl - sarcosyl - L - norvalyl - L - alanyl)amino] - äthylphosphonsäure vom Schmelzpunkt 233—236°C (Zers.); $[\alpha]_D^{20}$=—41,8°; $[\alpha]_{365}^{20}$=—152° (c=0,49% in Essigsäure).

12.2.2 Analog zu Beispiel 5.1.3 erhielt man aus dem Monobenzylaminsalz von (1R) - 1 - [(N - Benzyloxycarbonyl - sarcosyl - L - norvalyl - L - alanyl) - amino] - äthylphosphonsäure die (1R) - 1 - (Sarcosyl - L - norvalyl - L - alanylamino) - äthylphosphonsäure vom Schmelzpunkt 277—279°C (Zers.); $[\alpha]_D^{20}$=—96,0°; $[\alpha]_{365}^{20}$=—348° (c=0,51% in Wasser).

Beispiel 13

13.1 Herstellung des Ausgangsmaterials

13.1.1 Analog zu Beispiel 7.1.1.2 erhielt man aus N - Benzyloxycarbonyl - L - nitroarginin und (1R) - 1 - (Nitroarginylamino) - äthylphosphonsäure das Monobenzylaminsalz von (1R) - 1 - [(N - Benzyloxycarbonyl - L - nitroarginyl - L - nitroarginyl)amino] - äthylphosphonsäure vom Schmelzpunkt ca. 120°C (Zers.). Dieses Material wurde im nächsten Schritt ohne weitere Reinigung verwendet.

13.1.2 Analog zu Beispiel 5.1.3 erhielt man aus dem Monobenzylaminsalz von (1R) - 1 - [(N - Benzyloxycarbonyl - L - nitroarginyl - L - nitroarginyl)amino] - äthylphosphonsäure die (1R) - 1 - (L - Nitroarginyl - L - nitroarginylamino) - äthylphosphonsäure vom Schmelzpunkt ca. 195°C (Zers.). Umkristallisation einer Probe aus Wasser/Aethanol ergab 0,29 g eines Produkts vom Schmelzpunkt ca. 203°C (Zers.); $[\alpha]_D^{20}$=—5,3°; $[\alpha]_{365}^{20}$=—9,7°, (c=0,51% in Wasser.

13.2 Das Verfahren

13.2.1 Analog zu Beispiel 7.2.1 erhielt man aus der Vorratslösung von N - Benzyloxycarbonyl - sarcosin - N - hydroxysuccinimid - ester und (1R) - 1 - (L - Nitroarginyl - L - nitroarginylamino) - äthylphosphonsäure, nach Behandlung des nach Eindampfen des sauren Eluats erhaltenen Rückstands zuerst mit Aether dann mit Aceton, (1R) - 1 - [(Benzyloxycarbonyl - sarcosyl - L - nitroarginyl - L - nitroarginyl)amino] - äthylphosphonsäure vom Schmelzpunkt 185—189°C (Zers.).

13.2.2 Analog zu Beispiel 7.1.2 erhielt man aus (1R) - 1 - [(N - Benzyloxycarbonyl - sarcosyl - L - nitroarginyl - L - nitroarginyl)amino] - äthylphosphonsäure die (1R) - 1 - (Sarcosyl - L - nitroarginyl - L - nitroarginylamino) - äthylphosphonsäure vom Schmelzpunkt 228—230°C (Zers.); $[\alpha]_D^{20}$=—34,3°; $[\alpha]_{365}^{20}$=—117° (c=0,51%, in frisch hergestelltem 0,1N Natriumhydroxid).

13.2.3 Analog zu Beispiel 7.2.3 erhielt man aus (1R) - 1 - (Sarcosyl - L - nitroarginyl - L - nitroarginyl - amino) - äthylphosphonsäure die (1R) - 1 - (Sarcosyl - L - arginyl - L - arginylamino) - äthylphosphonsäure vom Schmelzpunkt ca. 220°C (Zers.); $[\alpha]_D^{20}$=—42,4°; $[\alpha]_{365}^{20}$=—160° (c=0,43% in Wasser).

Beispiel 14

14.1 Herstellung des Ausgangsmaterials

14.1.1 Analog zu Beispiel 7.1.1.2 erhielt man aus N - Benzyloxycarbonyl - L - norvalin - N - hydroxysuccinimid - ester und (1R) - 1 - (L - Nitroarginylamino) - äthylphosphonsäure, nach Verdampfung des sauren Eluats und Behandlung des Rückstands zuerst mit Aether und dann mit Aceton, die (1R) - 1 - [(N - Benzyloxycarbonyl - L - norvalyl - L - nitroarginyl)amino] - äthylphosphonsäure vom Schmelzpunkt 202—205°C (Zers.).

14.1.2 Analog zu Beispiel 7.1.2 erhielt man aus (1R) - 1 - [(N - Benzyloxycarbonyl - L - norvalyl - L - nitroarginyl) - amino] - äthylphosphonsäure die (1R) - 1 - (L - Norvalyl - L - nitroarginylamino) - äthylphosphonsäure vom Schmelzpunkt 267—269°C (Zers.); $[\alpha]_D^{20}$=—23,0°; $[\alpha]_{365}^{20}$=—84,0° (c=0,57%, frisch hergestellt in 0,1N Natriumhydroxid).

14.2 Das Verfahren

14.2.1 Analog zu Beispiel 7.2.1 erhielt man aus der Vorratslösung von N - Benzyloxycarbonyl - sarcosin - N - hydroxysuccinimid - ester und (1R) - 1 - (L - Norvalyl - L - nitroarginylamino) - äthylphosphonsäure das Monobenzylaminsalz von (1R) - 1 - [(N - Benzyloxycarbonyl - sarcosyl - L - norvalyl - L - nitroarginyl)amino] - äthylphosphonsäure vom Schmelzpunkt 230—235°C (Zers.).

14.2.2 Analog zu Beispiel 7.1.2 erhielt man aus dem Monobenzylaminsalz von (1R) - 1 - [(N - Benzyloxycarbonyl - sarcosyl - L - norvalyl - L - nitroarginyl) - amino] - äthylphosphonsäure die (1R) - 1 - (Sarcosyl - L - norvalyl - L - nitroarginylamino) - äthylphosphonsäure vom Schmelzpunkt 246—249°C (Zers.); $[\alpha]_D^{20}$=—47,4°; $[\alpha]_{365}^{20}$=—157° (c=53% in Wasser).

14.2.3 Analog zu Beispiel 7.2.3 erhielt man aus (1R) - 1 - (Sarcosyl - L - norvalyl - L - nitroarginylamino) - äthylphosphonsäure die (1R) - 1 - (Sarcosyl - L - norvalyl - L - arginylamino) - äthylphosphonsäure vom Schmelzpunkt ca. 200°C; $[\alpha]_D^{20}$=—68,1°; $[\alpha]_{365}^{20}$=—251° (c=0,52% in Wasser).

Beispiel 15

15.1 Herstellung des Ausgangsmaterials

15.1.1 Analog zu Beispiel 1.2.1 erhielt man aus N-Benzyloxycarbonyl - L - norvalin - N - hydroxysuccinimid - ester und (1R) - 1 - (L - Norvalylamino) - äthylphosphonsäure das Monobenzylaminsalz von (1R) - 1 - [(N - Benzyloxycarbonyl - L - norvalyl - L - norvalyl)amino] - äthylphosphonsäure vom Schmelzpunkt 243—245°C (Zers.). Eine Probe wurde aus warmem Wasser umkristallisiert. Das reine Monobenzylaminsalz schmolz bei 247—249°C (Zers.); $[\alpha]_D^{20}$=—35,7°; $[\alpha]_{365}^{20}$=—125° (c=0,46% in Essigsäure).

15.1.2 Analog zu Beispiel 5.1.3 erhielt man aus dem Monobenzylaminsalz von (1R) - 1 - [(N - Benzyloxycarbonyl - L - norvalyl - L - norvalyl)amino] - äthylphosphonsäure die (1R) - 1 - (L - Norvalyl - L - norvalylamino) - äthylphosphonsäure vom Schmelzpunkt 273—275°C (Zers.); $[\alpha]_D^{20}$=—36,0°; $[\alpha]_{365}^{20}$=—153°, (c=0,5% frisch hergestellt in 0,1N Natriumhydroxid).

15.2 Das Verfahren

15.2.1 Analog zu Beispiel 1.2.1 erhielt man aus der Vorratslösung von N - Benzyloxycarbonyl - sarcosin - N - hydroxysuccinimid - ester und (1R) - 1 - (L - Norvalyl - L - norvalylamino) - äthylphosphonsäure, nach Eindampfung das rohe Umsetzungsprodukt. Dieses Produkt war in wässrigem Methanol zu wenig löslich für den üblichen Ionenaustausch und wurde deshalb mit Wasser und 2N Salzsäure behandelt. Das Gemisch wurde bei Raumtemperatur gerührt und der Niederschlag abfiltriert. Das erhaltene Rohprodukt schmolz bei 210—214°C (Zers.). Nach Rühren mit Aceton erhielt man (1R) - 1 - [(N - Benzyloxycarbonyl - sarcosyl - L - norvalyl - L - norvalyl)amino] - äthylphosphonsäure vom Schmelzpunkt 217—219°C (Zers.); $[\alpha]_D^{20}$=—30,2°; $[\alpha]_{365}^{20}$=—113° (c=0,48% in Dimethylsulfoxid).

15.2.2 Analog zu Beispiel 5.1.3 erhielt man aus (1R) - 1 - [(N - Benzyloxycarbonyl - sarcosyl - L - norvalyl - L - norvalyl)amino] - äthylphosphonsäure die (1R) - 1 - (Sarcosyl - L - norvalyl - L - norvalylamino) - äthylphosphonsäure vom Schmelzpunkt 275—277°C (Zers.); $[\alpha]_D^{20}$=—83,5°; $[\alpha]_{365}^{20}$=—306° (c=0,3% in Wasser).

Beispiel 16

16.1 Herstellung des Ausgangsmaterials

16.1.1 Analog zu Beispiel 1.2.1 erhielt man aus N - Benzyloxycarbonyl - glycin - N - hydroxysuccinimid - ester und (1R) - 1 - (L - Norvalylamino) - äthylphosphonsäure das Monobenzylaminsalz von (1R) - 1 - [(N - Benzyloxycarbonyl - glycyl - L - norvalyl)amino] - äthylphosphonsäure vom Schmelzpunkt 217—220°C (Zers.).

16.1.2 Analog zu Beispiel 5.1.3 erhielt man aus dem Monobenzylaminsalz von (1R) - 1 - [(N - Benzyloxycarbonyl - glycyl - L - norvalyl)amino] - äthylphosphonsäure die (1R) - 1 - (Glycyl - L - norvalylamino) - äthylphosphonsäure vom Schmelzpunkt 265—267°C (Zers.); $[\alpha]_D^{20}$=—74,2°; $[\alpha]_{365}^{20}$=—271° (c=0,49% in Wasser).

16.2 Das Verfahren

16.2.1 Analog zu Beispiel 1.2.1 erhielt man aus N - Benzyloxycarbonyl - sarcosin - N - hydroxysuccinimid - ester und (1R) - 1 - (Glycyl - L - norvalylamino) - äthylphosphonsäure das Monobenzylaminsalz von (1R) - 1 - [(N - Benzyloxycarbonyl - sarcosyl - glycyl - L - norvalyl)amino] - äthylphosphonsäure vom Schmelzpunkt 225—230°C (Zers.).

16.2.2 Analog zu Beispiel 5.1.3 erhielt man aus dem Monobenzylaminsalz von (1R) - 1 - [(N - Benzyloxycarbonyl - sarcosyl - glycyl - L - norvalyl)amino] - äthylphosphonsäure die (1R) - 1 - (Sarcosyl - glycyl - L - norvalylamino) - äthylphosphonsäure vom Schmelzpunkt 255—257°C (Zers.): $[\alpha]_D^{20}$=—64,2°; $[\alpha]_{365}^{20}$=—235° (c=0,49% in Wasser).

Beispiel 17

17.1 Herstellung des Ausgangsmaterials

17.1.1 Analog zu Beispiel 7.1.1.2 erhielt man aus N - Benzyloxycarbonyl - L - nitroarginin und (1R) - 1 - [L - norvalylamino] - äthylphosphonsäure das Monobenzylaminsalz von (1R) - 1 - [(N - Benzyloxycarbonyl - L - nitroarginyl - L - norvalyl)amino] - äthylphosphonsäure vom Schmelzpunkt 191—196°C (Zers.), welches direkt im nächsten Schritt verwendet wurde.

17.1.2 Analog zu Beispiel 7.1.2 erhielt man aus dem Monobenzylaminsalz von (1R) - 1 - [(N - Benzyloxycarbonyl - L - nitroarginyl - L - norvalyl)amino] - äthylphosphonsäure die (1R) - 1 - (L - Nitroarginyl - L - norvalylamino) - äthylphosphonsäure vom Schmelzpunkt 240—242°C (Zers.); $[\alpha]_D^{20}$=—14,0°; $[\alpha]_{365}^{20}$=—46,7 (c=0,49% in Wasser).

17.2 Das Verfahren

17.2.1 Analog zu Beispiel 7.2.1 erhielt man aus der Vorratslösung von N - Benzyloxycarbonyl -

sarcosin - N - hydroxysuccinimid - ester und (1R) - 1 - (L - Nitroarginyl - L - norvalylamino) - äthylphosphonsäure das Monobenzylaminsalz von (1R) - 1 - [(N - Benzyloxycarbonyl - sarcosyl - L - nitroarginyl - L - norvalyl)amino] - äthylphosphonsäure vom Schmelzpunkt 208—212°C (Zers.), welches im nächsten Schritt verwendet wurde.

17.2.2 Analog zu Beispiel 7.1.2 erhielt man aus dem Monobenzylaminsalz von (1R) - 1 - [(N - Benzyloxycarbonyl - sarcosyl - L - nitroarginyl - L - norvalyl) - amino] - äthylphosphonsäure die (1R) - 1 - (Sarcosyl - L - nitroarginyl - L - norvalylamino) - äthylphosphonsäure vom Schmelzpunkt 245—250°C (Zers.); $[\alpha]_D^{20}$=—51,4°; $[\alpha]_{365}^{20}$=—179° (c=0,49% frisch hergestellt in 0,1N Natriumhydroxid).

17.2.3 Analog zu Beispiel 7.2.3 erhielt man aus (1R) - 1 - (Sarcosyl - L - nitroarginyl - L - norvalylamino) - äthylphosphonsäure die (1R) - 1 - (Sarcosyl - L - arginyl - L - norvalylamino) - äthylphosphonsäure vom Schmelzpunkt ca. 220°C (Zers.); $[\alpha]_D^{20}$=—56,9°; $[\alpha]_{365}^{20}$=—223° (c=0,51% in Wasser).

Beispiel 18

18.1 Herstellung des Ausgangsmaterials

18.1.1.1 Analog zu Beispiel 7.1.1.1 erhielt man aus N - Benzyloxycarbonyl - L - valin und (1R) - 1 - [L - Norvalylamino] - äthylphosphonsäure die (1R) - 1 - [(N - Benzyloxycarbonyl - L - valyl - L - norvalyl) - amino] - äthylphosphonsäure zusammen mit (1R) - 1 - [(N - Isobutoxycarbonyl - L - norvalyl)amino] - äthylphosphonsäure, gemäss NMR Spektrum. Dieses Material wurde im nächsten Schritt ohne weitere Reinigung verwendet.

18.1.1.2 Analog zu Beispiel 1.2.1 erhielt man aus (1R) - 1 - (L - Norvalylamino) - äthylphosphonsäure und N - Benzyloxycarbonyl - L - valin - N - hydroxysuccinimid - ester, nach Verdampfung der Lösungsmittel, einen Rückstand, welcher sich nicht in Wasser löste. Die wässrige Suspension wurde gerührt, dann 2N HCl zugefügt bis zu einem pH unter 1. Der erhaltene gelatinöse Niederschlag wurde bei 0°C über Nacht stehengelassen. Der Niederschlag wurde abfiltriert und getrocknet, wobei man einen wachsartigen Festkörper erhielt. Dieser wurde mit Aceton gerührt und filtriert. Man erhielt so rohe (1R) - 1 - [(N - Benzyloxycarbonyl - L - valyl - L - norvalyl)amino] - äthylphosphonsäure vom Schmelzpunkt 205—210°C (Zers.).

18.1.2.1 Analog zu Beispiel 7.1.2 erhielt man aus roher (1R) - 1 - [(N - Benzyloxycarbonyl - L - valyl - L - norvalyl) - amino] - äthylphosphonsäure (hergestellt wie sub 18.1.1.1 beschrieben) (1R) - 1 - (L - Valyl - L - norvalylamino) - äthylphosphonsäure vom Schmelzpunkt 268—270°C (Zers.); $[\alpha]_D^{20}$=—33,4°; $[\alpha]_{365}^{20}$=—110° (c=0,47% in 1N Salzsäure).

18.1.2.2 Analog zu Beispiel 7.1.2 erhielt man aus roher (1R) - 1 - [(N - Benzyloxycarbonyl - L - valyl - L - norvalyl) - amino] - äthylphosphonsäure (hergestellt wie sub 18.1.1.2 beschrieben) (1R) - 1 - (L - Valyl - L - norvalylamino) - äthylphosphonsäure vom Schmelzpunkt 268—270°C (Zers.); $[\alpha]_D^{20}$=—34,6°; $[\alpha]_{365}^{20}$=—114° (c=0,54% in 1N Salzsäure).

18.2 Das Verfahren

18.2.1 Analog zu Beispiel 1.2.1 erhielt man aus (1R) - 1 - (L - Valyl - L - norvalylamino) - äthylphosphonsäure und N - Benzyloxycarbonyl - sarcosin - N - hydroxysuccinimid - ester die (1R) - 1 - [(N - Benzyloxycarbonyl - sarcosyl - L - valyl - L - norvalyl)amino] - äthylphosphonsäure vom Schmelzpunkt 228—230°C (Zers.).

18.2.2 Analog zu Beispiel 5.1.3 erhielt man aus (1R) - 1 - [(N - Benzyloxycarbonyl - sarcosyl - L - valyl - L - norvalyl)amino] - äthylphosphonsäure die (1R) - 1 - (Sarcosyl - L - valyl - L - norvalylamino) - äthylphosphonsäure vom Schmelzpunkt 280—282°C (Zers.); $[\alpha]_D^{20}$=—90,4°; $[\alpha]_{365}^{20}$=—329° (c=0,52% in Wasser).

Beispiel 19

19.1 Herstellung des Ausgangsmaterials

19.1.1 Analog zu J. Org. Chem. *35,* 1914 (1970), wurden 10,45 g (50 mMol) N - Benzyloxycarbonyl - glycin in 100 ml trockenem Dimethylformamid bei Raumtemperatur gerührt und mit 62,4 g (400 mMol) Aethyl jodid und ca. 46 g (180 mMol) Silberoxid behandelt.

Das Gemisch wurde über Nacht gerührt und filtriert und das Filtrat mit 400 ml Methylenchlorid verdünnt und über Nacht gekühlt. Das ausgeschiedene Silberjodid wurde abfiltriert und das Filtrat gewaschen (2×mit je 100 ml wässriger Kaliumcyanidlösung und dann 3×mit je 150 ml Wasser). Die organische Schicht wurde über Natriumsulfat getrocknet, filtriert und eingedampft, zuerst am Wasserstrahlvakuum und dann am Oelpumpenvakuum. Man erhielt so 14,25 g rohes Aethyl - N - Benzyloxycarbonyl - N - äthyl - glycinat in Form eines gelben Oels.

19.1.2 Auf die in der vorgenannten Literatur beschriebene Art wurden 14,25 g (ca. 50 mMol) rohes Aethyl - N - benzyloxycarbonyl - N - äthyl - glycinat bei Raumteperatur mit 150 ml Aethanol gerührt und dann mit 50 ml 1N wässrigem Natriumhydroxid behandelt. Die Mischung wurde dreissig Minuten weitergerührt. Die Lösungsmittel wurden abgedampft und das restliche Oel wurde in 150 ml Wasser gelöst, auf 0°C gekühlt und dann mit ca. 30 ml 2N HCl auf pH 2 angesäuert (Congorot). Der resultierende oelige Niederschlag wurde mit Aethylacetat (2×200 ml) extrahiert und die organische

Phase mit Wasser gewaschen. Die vereinigten Aethylacetatextrakte wurden über Natriumsulfat getrocknet, filtriert und zu einem Oel eingedampft. Verreiben mit Tetrachlorkohlenstoff ergab eine Festsubstanz, welche abfiltriert wurde. Nach Eindampfen des Filtrats erhielt man ca. 9,5 g N - Benzyloxycarbonyl - N - äthyl - glycin in Form eines Oels, welches ein befriedigendes NMR-Spektrum aufwies. Dieses Produkt wurde im nächsten Schritt ohne weitere Reinigung verwendet.

19.1.3 Analog zu Beispiel 1.1.2 erhielt man aus N - Benzyloxycarbonyl - N - äthyl - glycin den N - Benzyloxycarbonyl - N - äthyl - glycin - N - hydroxysuccinimid - ester in Form eines Oels, welches in Dimethylformamid gelöst und ohne weitere Reinigung im nächsten Schritt verwendet wurde.

19.2 Das Verfahren

19.2.1 Analog zu Beispiel 1.2.1 erhielt man aus der vorerwähnten Lösung von N - Benzyloxycarbonyl - N - äthyl - glycin - N - hydroxysuccinimid - esterund (1R) - 1 - (L - Alanyl - L - alanylamino) - äthylphosphonsäure ein saures Eluat aus dem Harz. Dieses Eluat wurde zu einem Gummi eingedampft, welcher zwischen Wasser und Methylenchlorid verteilt wurde. Eine wesentliche Menge eines unlöslichen Festkörpers wurde erhalten, welcher abfiltriert und getrocknet wurde. Das so erhaltene rohe Produkt schmolz bei 186—189°C (Zers.). Umkristallisation aus Aethanol ergab (1R) - 1 - [(N - Benzyloxycarbonyl - N - äthyl - glycyl - L - alanyl - L - alanyl)amino] - äthylphosphonsäure vom Schmelzpunkt 180—181°C (Zers.); $[\alpha]_D^{20}=24,5°$; $[\alpha]_{365}^{20}=-101°$ (c=0,5% in Dimethylformamid).

Das vorerwähnte Methylenchlorid/Wasserfiltrat wurde in eine organische und eine wässrige Phase getrennt und die wässrige Phase mit Methylenchlorid (250 ml) gewaschen. Die wässrige Schicht wurde dann mit Benzylamin bis zu einem pH von 4,5 titriert. Man erhielt so das Monobenzylaminsalz von (1R) - 1 - [(N - Benzyloxycarbonyl - N - äthyl - glycyl - L - alanyl - L - alanyl)amino] - äthylphosphonsäure vom Schmelzpunkt 219—223°C (Zers.); $[\alpha]_D^{20}=-67,6°$; $[\alpha]_{365}^{20}=-236°$ (c=0,5% in Wasser).

19.2.2 (1R) - 1 - [(N - Benzyloxycarbonyl - N - äthyl - glycyl - L - alanyl)amino] - äthylphosphonsäure wurde in Wasser gelöst und 10%iger Palladiumkohle-Katalysator, Methanol und einigen Tropfen Essigsäure wurden zugefügt. Das Gemisch wurde bei Raumtemperatur und Normaldruck über Nacht hydriert. Der Katalysator wurde abfiltriert und das Filtrat eingedampft. Der feste Rückstand wurde aus Wasser/Aethanol umkristallisiert und ergab (1R) - 1 - (N - Aethyl - glycyl - L - alanyl - L - alanylamino) - äthylphosphonsäure vom Schmelzpunkt 267—270°C (Zers.); $[\alpha]_D^{20}=-421°$ (c=0,5% in Wasser).

Beispiel 20

20.1 Herstellung des Ausgangsmaterials

20.1.1 Analog zu Beispiel 19.1.1 erhielt man aus N - Benzyloxycarbonyl - glycin, Allyljodid und Silberoxid in trockenem Dimethylformamid rohes Allyl - N - allyl - N - benzyloxycarbonyl - glycinat in Form eines gelben Oels.

20.1.2 Analog zu Beispiel 19.1.2 erhielt man aus Allyl - N - allyl - N - benzyloxycarbonyl - glycinat das N - Allyl - N - benzyloxycarbonyl - glycin in Form eines Oels.

20.1.3 Analog zu Beispiel 1.1.2 erhielt man aus N - Allyl - N - benzyloxycarbonyl - glycin den N - Allyl - N - benzyloxycarbonyl - glycin - N - hydroxysuccinimid - ester in Form eines gelben Oels, welches in 25 ml Dimethylformamide gelöst und im nächsten Schritt ohne weitere Reinigung verwendet wurde.

20.2 Das Verfahren

20.2.1 Analog zu Beispiel 1.2.1 erhielt man aus der vorerwähnten Lösung von N - Allyl - N - benzyloxycarbonyl - glycin - N - hydroxysuccinimid - ester und (1R) - 1 - (L - Alanyl - L - alanylamino) - äthylphosphonsäure (aus der Wasser/Methylenchloridverteilung des Gummis aus dem sauren Eluat), eine Festsubstanz, welche aufgrund der Dünnschichtchromatographie, reine (1R) - 1 - [(N - Allyl - N - benzyloxycarbonyl - glycyl - L - alanyl - L - alanyl)amino] - äthylphosphonsäure zu sein schien.

Das vorerwähnte Wasser/Methylenchloridfiltrat wurde in zwei Schichten getrennt. Die wässrige Schicht wurde mit Methylenchlorid gewaschen und dann eingedampft. Man erhielt einen Gummi, welcher in Aethanol/Wasser gelöst und in das Benzylaminsalz übergeführt wurde. Eindampfen ergab einen festen Gummi, welcher aus 90% Aethanol-Aether umkristallisiert wurde. Man erhielt so das Monobenzylaminsalz von (1R) - 1 - [(N - Allyl - N - benzyloxycarbonyl - glycyl - L - alanyl - L - alanyl)amino] - äthylphosphonsäure vom Schmelzpunkt 233—235°C (Zers.): $[\alpha]_D^{20}=-45,1°$; $[\alpha]_{365}^{20}=-170°$ (c=0,5% in Essigsäure).

20.2.2 Analog zu Beispiel 1.2.2 erhielt man aus dem Monobenzylaminsalz von (1R) - 1 - [(N - Allyl - N - benzyloxycarbonyl - glycyl - L - alanyl - L - alanyl)amino] - äthylphosphonsäure (jedoch unter Verwendung von Wasser/Aethanol (1:1) zur Gewinnung der freien Säure im Ionenaustauschschritt), nach Hydrogenolyse der N - Benzyloxycarbonyl - Gruppe auf übliche Weise, aber mit gleichzeitiger Reduktion der Allyl-Gruppe zur n-Propyl-Gruppe, das Rohprodukt in Form eines Gels. Kristallisation aus Wasser/Aethanol ergab (1R) - 1 - [N - (n - Propyl) - glycyl - L - alanyl - L - alanylamino] - äthylphosphonsäure vom Schmelzpunkt 263—264°C (Zers.); $[\alpha]_D^{20}$=—110°; $[\alpha]_{365}^{20}$=—392° (c=1% in Wasser).

Beispiel 21

Analog zu Beispiel 7.1.2 erhielt man aus dem Monobenzylaminsalz von (1R) - 1 - [(N - Allyl - N - benzyloxycarbonyl - glycyl - L - alanyl - L - alanyl)amino] - äthylphosphonsäure (hergestellt wie in Beispiel 20.2.1 beschrieben) die (1R) - 1 - (N - Allyl - glycyl - L - alanyl - L - alanylamino) - äthylphosphonsäure vom Schmelzpunkt 251—253°C (Zers.); $[\alpha]_D^{20}$=—110°; $[\alpha]_{365}^{20}$=—394° (c=1% in Wasser).

Beispiel 22

22.1 Herstellung des Ausgangsmaterials

22.1.1 Analog zu Beispiel 19.1.1 erhielt man aus N - Benzyloxycarbonyl - glycin, n-Hexyljodid und Silberoxid in trockenem Dimethylformamid rohes n - Hexyl - N - benzyloxycarbonyl - N - (n - hexyl) - glycinat in Form eines Oels.

22.1.2 Analog zu Beispiel 19.1.2 erhielt man aus n - Hexyl - N - benzyloxycarbonyl - N - (n - hexyl) - glycinat das N - Benzyloxycarbonyl - N - (n - hexyl) - glycin in Form eines Oels.

22.1.3 Analog zu Beispiel 1.1.2 erhielt man aus N - Benzyloxycarbonyl - N - (n - hexyl) - glycin den N - Benzyloxycarbonyl - N - (n - hexyl) - glycin - N - hydroxysuccinimid - ester in Form eines gelben Oels, welches in Dimethylformamid gelöst und ohne weitere Reinigung im nächsten Schritt verwendet wurde.

22.2 Das Verfahren

22.2.1 Analog zu Beispiel 1.2.1 erhielt man aus der vorerwähnten Lösung von N - Benzyloxycarbonyl - N - (n - hexyl) - glycin - N - hydroxysuccinimid - ester und (1R) - 1 - (L - Alanyl - L - alanylamino) - äthylphosphonsäure nach Behandlung mit dem Ionenaustauscherharz ein saures Eluat. Das Eluat wurde zu einer Festsubstanz eingedampft, welche zwischen Wasser und Aethylacetat verteilt wurde. Die Schichten wurden getrennt, die Wässrige Phase wurde mit Aethylacetat gewaschen und die organischen Phasen wurden separat mit Wasser gewaschen. Die vereinigten Aethylacetatextrakte wurden über Natriumsulfat getrocknet und zu einem gelben Festkörper eingedampf, welcher mit Aether verrieben und filtriert wurde. Man erhielt eīn hygroskopisches Rohprodukt vom Schmelzpunkt 183—185°C (Zers.). Umkristallisation aus Aethylacetat/Aether ergab (1R) - [(N - Benzyloxycarbonyl - N - (n - hexyl) - glycyl - L - alanyl - L - alanyl)amino] - äthylphosphonsäure vom Schmelzpunkt 177—179°C (Zers.); $[\alpha]_D^{20}$=—46,1°; $[\alpha]_{365}^{20}$=—173° (c=0,5% in Essigsäure).

22.2.2 Analog zu Beispiel 5.1.3 erhielt man aus (1R) - 1 - [(N - Benzyloxycarbonyl - N - (n - hexyl) - glycyl - L - alanyl - L - alanyl)amino] - äthylphosphonsäure die (1R) - 1 - (N - (n - Hexyl) - glycyl - L - alanyl - L - alanylamino) - äthylphosphonsäure vom Schmelzpunkt 262—265°C (Zers.); $[\alpha]_D^{20}$=—88,2°; $[\alpha]_{365}^{20}$=—323° (c=0,5% in frisch hergestelltem 0,1N Natriumhydroxid).

Beispiel 23

23.1 Herstellung des Ausgangsmaterials

23.1.1 Analog zu Beispiel 1.1.2 erhielt man aus N - Benzyloxycarbonyl - N - methyl - L - alanin (hergestellt gemäss J. Org. Chem. *35,* (1970), 1915) den N - Benzyloxycarbonyl - N - methyl - L - alanin - N - hydroxysuccinimid - ester in Form eines Oels.

23.2 Das Verfahren

23.2.1 Analog zu Beispiel 1.2.1 erhielt man aus N - Benzyloxycarbonyl - N - methyl - L - alanin - N - hydroxysuccinimid - ester in Dimethylformamid und (1R) - 1 - (L - Alanyl - L - alanyl - amino) - äthylphosphonsäure aus dem Kationenaustauscherharz ein saures Eluat. Eindampfung dieses Eluats ergab einen Festkörper, welcher mit Wasser trituriert und dann abfiltriert wurde. Umkristallisation aus Aethanol ergab (1R) - 1 - [(N - Benzyloxycarbonyl - N - methyl - L - alanyl - L - alanyl - L - alanyl)amino] - äthylphosphonsäure vom Schmelzpunkt 225—226°C (Zers.); $[\alpha]_D^{20}$=—99,3°; $[\alpha]_{365}^{20}$=—348° (c=0,5% in Wasser).

23.2.2 Analog zu Beispiel 19.2.2 erhielt man aus (1R) - 1 - [(N - Benzyloxycarbonyl - N - methyl - L - alanyl - L - alanyl - L - alanyl)amino] - äthylphosphonsäure (jedoch unter Hydrierung in wässrigem Aethanol statt in wässrigem Methanol) die (1R) - 1 - (N - Methyl - L - alanyl - L - alanyl - L - alanylamino) - äthylphosphonsäure vom Schmelzpunkt 318—320°C (Zers.); $[\alpha]_D^{20}$=—121°; $[\alpha]_{365}^{20}$=—418° (c=0,5% in Wasser).

Beispiel 24

24.1 Herstellung des Ausgangsmaterials

24.1.1 Analog zu Beispiel 19.1.1 erhielt man aus N - Benzyloxycarbonyl - L - norvalin, Methyljodid und Silberoxid in trockenem Dimethylformamid rohes Methyl - N - benzyloxycarbonyl - N - methyl - L - norvalinat.

24.1.2 Analog zu Beispiel 19.1.2 erhielt man aus Methyl - N - benzyloxycarbonyl - N - methyl - L - norvalinat das N - Benzyloxycarbonyl - N - methyl - L - norvalin in Form eines Oels.

24.1.3 Analog zu Beispiel 1.1.2 erhielt man aus N - Benzyloxycarbonyl - N - methyl - L - norvalin den N - Benzyloxycarbonyl - N - methyl - L - norvalin - N - hydroxysuccinimid - ester in Form eines Oels, welches in Dimethylformamid gelöst und im nächsten Schritt verwendet wurde.

24.2 Das Verfahren

24.2.1 Analog zu Beispiel 1.2.1 erhielt man aus der gemäss 24.1.3 hergestellten Lösung und (1R) - 1 - (L - Norvalyl - L - norvalylamino) - äthylphosphonsäure (hergestellt gemäss Beispiel 15.1.2) die (1R) - 1 - [(N - Benzyloxycarbonyl - N - methyl - L - norvalyl - L - norvalyl - L - norvalyl)amino] - äthylphosphonsäure vom Schmelzpunkt 195—200°C (Zers.); $[\alpha]_D^{20}$=—28,7°; $[\alpha]_{365}^{20}$=—116° (c=0,5% in Dimethylformamid).

24.2.2 Analog zu Beispiel 5.1.3 erhielt man aus (1R) - 1 - [(N - Benzyloxycarbonyl - N - methyl - L - norvalyl - L - norvalyl - L - norvalyl)amino] - äthylphosphonsäure die (1R) - 1 - (N - Methyl - L - norvalyl - L - norvalyl - L - norvalylamino) - äthylphosphonsäure vom Schmelzpunkt 307—309°C (Zers.); $[\alpha]_D^{20}$=—81,1°; $[\alpha]_{365}^{20}$=—291°C (c=0,5% in frisch hergestelltem 0,1N Natriumhydroxid).

Beispiel 25

25.1 Herstellung des Ausgangsmaterials

25.1.1 Analog zu Beispiel 19.1.1 erhielt man aus N - Benzyloxycarbonyl - L - valin, Methyljodid und Silberoxid in trockenem Dimethylformamid das Methyl - N - benzyloxycarbonyl - N - methyl - L - valinat in Form eines Oels.

25.1.2 In Analogie zu Beispiel 19.1.2 erhielt man aus Methyl - N - benzyloxycarbonyl - N - methyl - L - valinat rohes N - Benzyloxycarbonyl - N - methyl - L - valin, welches im nächsten Schritt ohne weitere Reinigung verwendet wurde. Schmelzpunkt der reinen Verbindung: 64—65°C; $[\alpha]_{365}^{20}$=—318° (c=0,98% in Aethanol).

25.1.3 Analog zu Beispiel 1.1.2 erhielt man aus rohem N - Benzyloxycarbonyl - N - methyl - L - valin den N - Benzyloxycarbonyl - N - methyl - L - valin - N - hydroxysuccinimid - ester in Form eines Oels, welches in Dimethylformamid aufgenommen und im nächsten Schritt verwendet wurde.

25.2 Das Verfahren

25.2.1 Analog zu Beispiel 1.2.1 erhielt man aus der vorerwähnten Lösung von N - Benzyloxycarbonyl - N - methyl - L - valin - N - hydroxysuccinimid - ester und (1R) - 1 - (L - Valyl - L - norvalylamino) - äthylphosphonsäure (hergestellt wie im Beispiel 18 beschrieben), nach Verdampfung und Behandlung mit, 0,2N Salzsäure, einen Gummi. Dieser Gummi wurde mit Methylenchlorid behandelt. Zur erhaltenen Emulsion wurde Methanol zugefügt. Es entstanden zwei Schichten. Die zwei Schichten wurden verdampft und der erhaltene Gummi wurde mit Aether, dann mit Wasser und schliesslich mit Aceton behandelt. Man erhielt (1R) - 1 - [(N - Benzyloxycarbonyl - N - methyl - L - valyl - L - valyl - L - norvalyl)amino] - äthylphosphonsäure vom Schmelzpunkt 194—197°C (Zers.).

25.2.2 Analog zu Beispiel 5.1.3 erhielt man aus (1R) - 1 - [(N - Benzyloxycarbonyl - N - methyl - L - valyl - L - valyl - L - norvalyl)amino] - äthylphosphonsäure die (1R) - 1 - (N - Methyl - L - valyl - L - valyl - L - norvalyl)amino] - äthylphosphonsäure vom Schmelzpunkt 309—312°C (Zers.); $[\alpha]_D^{20}$=—55,1°; $[\alpha]_{365}^{20}$=—215° (c=0,56% in Trifluoressigsäure).

Beispiel 26

26.1 Herstellung des Ausgangsmaterials

26.1.1 Analog zu Beispiel 19.1.1 erhielt man aus N - Benzyloxycarbonyl - L - leucin, Methyljodid und Silberoxid in trockenem Dimethylformamid Methyl - N - benzyloxycarbonyl - N - methyl - L - leucinat in Form eines Oels.

26.1.2 Analog zu Beispiel 19.1.2 erhielt man aus Methyl - N - benzyloxycarbonyl - N - methyl - L - leucinat ein Oel, welches sich beim Stehen verfestigte. Verreiben mit Petroläther und Filtration ergaben N - Benzyloxycarbonyl - N - methyl - L - leucin vom Schmelzpunkt 69—71°C; $[\alpha]_D^{20}$=—22,0°; $[\alpha]_{365}^{20}$=—91,2° (c=0,5% in Aethylacetat).

26.1.3 Analog zu Beispiel 1.1.2 erhielt man aus N - Benzyloxycarbonyl - N - methyl - L - leucin Rohprodukt in Form eines Oels. Verreiben mit Petroläther ergab N - Benzyloxycarbonyl - N - methyl - L - leucin - N - hydroxysuccinimid - ester in Form einer Festsubstanz vom Schmelzpunkt 73—75°C; $[\alpha]_D^{20}$=—46,9°; $[\alpha]_{365}^{20}$=—178° (c=0,5% in Aethylacetat).

26.2 Das Verfahren

26.2.1 3,23 g (10 mMol) (1R) - 1 - (L - Norvalyl - L - norvalylamino) - äthylphosphonsäure (hergestellt gemäss Beispiel 15) wurden bei 0°C mit 30 ml Wasser, 2,8 ml (20 mMol) Triäthylamin und 60 ml Dimethylformamid gerührt. Zum Gemisch wurden 4,7 g (12,5 mMol) N - Benzyloxycarbonyl - N - methyl - L - leucin - N - hydroxysuccinimid - ester zugefügt. Das Gemisch wurde weitere zwei

Stunden bei 0°C gerührt, dann noch sechzehn Stunden bei Raumtemperatur, was eine heterogene Mischung ergab. Ein Feststoff wurde abfiltriert und das Filtrat eingedampft. Man erhielt 6 g eines Oels, welches mit 100 ml Wasser gerührt und dann mit 10 ml 2N Salzsäure versetzt wurde. Das Gemisch wurde zwei Stunden bei Raumtemperatur gerührt und dann mit 100 ml Methylenchlorid versetzt. Die Schichten wurden getrennt. Die wässrige Phase wurde mit Methylenchlorid extrahiert. Die organischen Extrakte wurden mit Wasser nachgewaschen, getrocknet und eingedampft. Man erhielt ca. 4,7 g eines Oels, welches mit Benzylamin behandelt wurde. Verdampfung und Behandlung mit Aceton ergaben 1,10 g Monobenzylaminsalz von (1R) - 1 - [(N - Benzyloxycarbonyl - N - methyl - L - leucyl - L - norvalyl - L - norvalyl) - amino] - äthylphosphonsäure vom Schmelzpunkt 215—218°C (Zers.).

26.2.2 Analog zu Beispiel 7.1.2 erhielt man aus (1R) - 1 - [(N - Benzyloxycarbonyl - N - methyl - L - leucyl - L - norvalyl - L - norvalyl)amino] - äthylphosphonsäure (1R) - 1 - (N - Methyl - L - leucyl - L - norvalyl - L - norvalyl)amino] - äthylphosphonsäure vom Schmelzpunkt 317—318°C (Zers.); $[\alpha]_D^{20}=-39,9°$; $[\alpha]_{365}^{20}=-159°$ (c=0,5% in Trifluoressigsäure).

Beispiel 27

27.1 Herstellung des Ausgangsmaterials

27.1.1 Analog zu Beispiel 15.2.1 erhielt man aus (1R) - 1 - (L - Norvalyl - L - norvalylamino) - äthylphosphonsäure (siehe Beispiel 15.1.2) und N - Benzyloxycarbonyl - glycin - N - hydroxysuccinimid - ester die (1R) - 1 - [(N - Benzyloxycarbonyl - glycyl - L - norvalyl - L - norvalyl)amino] - äthylphosphonsäure vom Schmelzpunkt 195—205°C (Zers.).

27.1.2 Analog zu Beispiel 5.1.3 erhielt man aus (1R) - 1 - [(N - Benzyloxycarbonyl - glycyl - L - norvalyl - L - norvalyl) - amino] - äthylphosphonsäure die (1R) - 1 - (Glycyl - L - norvalyl - L - norvalylamino) - äthylphosphonsäure vom Schmelzpunkt 268—270°C (Zers.); $[\alpha]_D^{20}=-83,1°$; $[\alpha]_{365}^{20}=-292°$ (c=0,4% 1N Salzsäure).

27.2 Das Verfahren

27.2.1 Analog zu Beispiel 15.2.1 erhielt man aus (1R) - 1 - (Glycyl - L - norvalyl - L - norvalylamino) - äthylphosphonsäure und einer Lösung von N - Benzyloxycarbonyl - sarcosin - N - hydroxysuccinimid - ester die (1R) - 1 - [(N - Benzyloxycarbonyl - sarcosyl - glycyl - L - norvalyl - L - norvalyl)amino] - äthylphosphonsäure vom Schmelzpunkt 210—215°C (Zers.).

27.2.2 Analog zu Beispiel 5.1.3 erhielt man aus (1R) - 1 - [(N - Benzyloxycarbonyl - sarcosyl - glycyl - L - norvalyl - L - norvalyl)amino]äthylphosphonsäure die (1R) - 1 - (Sarcosyl - glycyl - L - norvalyl - L - norvalylamino) - äthylphosphonsäure vom Schmelzpunkt 278—280°C (Zers.); $[\alpha]_D^{20}=-85,8°$; $[\alpha]_{365}^{20}=-312°$ (c=0,51% in Wasser).

Beispiel 28

28.1 Herstellung des Ausgangsmaterials

28.1.1 Analog zu Beispiel 15.2.1 erhielt man aus (1R) - 1 - (L - Valyl - L - norvalylamino) - äthylphosphonsäure (hergestellt gemäss Beispiel 18) und N - Benzyloxycarbonyl - L - valin - N - hydroxysuccinimid - ester (hergestellt gemäss JACS, *86*, (1964) 1839) die (1R) - 1 - [(N - Benzyloxycarbonyl - L - valyl - L - valyl - L - norvalyl)amino] - äthylphosphonsäure vom Schmelzpunkt 257—260°C (Zers.).

28.1.2 Analog zu Beispiel 5.1.3 erhielt man aus (1R) - 1 - [(N - Benzyloxycarbonyl - L - valyl - L - valyl - L - norvalylamino] - äthylphosphonsäure die (1R) - 1 - (L - Valyl - L - valyl - L - norvalylamino) - äthylphosphonsäure vom Schmelzpunkt ca. 280°C (Zers.); $[\alpha]_D^{20}=-70,9°$; $[\alpha]_{365}^{20}=-253°$ (c=0,48% in 1N Salzsäure).

28.2 Das Verfahren

28.2.1 Analog zu Beispiel 15.2.1 erhielt man aus (1R) - 1 - (L - Valyl - L - valyl - L - norvalylamino) - äthylphosphonsäure und einer Lösung von N - Benzyloxycarbonyl - sarcosin - N - hydroxysuccinimid - ester 2,14 g (1R) - 1 - [(N - Benzyloxycarbonyl - sarcosyl - L - valyl - L - valyl - L - norvalyl)amino] - äthylphosphonsäure vom Schmelzpunkt 257—261°C (Zers.).

28.2.2 Analog zu Beispiel 5.1.3 erhielt man aus (1R) - 1 - [(N - Benzyloxycarbonyl - sarcosyl - L - valyl - L - valyl - L - norvalyl)amino] - äthylphosphonsäure die (1R) - 1 - (Sarcosyl - L - valyl - L - valyl - L - norvalylamino) - äthylphosphonsäure vom Schmelzpunkt 292—294°C (Zers.); $[\alpha]_D^{20}=-87,9°$; $[\alpha]_{365}^{20}=-319°$ (c=0,33% in Trifluoressigsäure).

Beispiel 29

29.1 Herstellung des Ausgangsmaterials

29.1.1.1 Analog zu Beispiel 1.2.1 erhielt man aus N - Benzyloxycarbonyl - L - norvalin - N - hydroxysuccinimid - ester und Aminomethylphosphonsäure rohes Monobenzylaminsalz des gewünschten Produkts vom Schmelzpunkt 195—198°C (Zers.). Umkristallisation einer Probe aus heissem Wasser ergab reines Monobenzylaminsalz von (N - Benzyloxycarbonyl - L - norvalyl) - amino - methylphosphonsäure vom Schmelzpunkt 203—205°C (Zers.); $[\alpha]_D^{20}=-8,2°$; $[\alpha]_{365}^{20}=-21,3°$ (c=0,5% in Essigsäure).

29.1.1.2 45,4 g (180 mMol) N - Benzyloxycarbonyl - L - norvalin wurden in 200 ml Methylenchlorid gerührt, während 31,76 g (180 mMol) Dimethyl - aminomethylphosphonat - hydrochlorid zugefügt wurden. Die resultierende Suspension wurde auf —12°C gekühlt, worauf 25,3 ml (180 mMol) trockenes Triäthylamin tropfenweise zugefügt wurden. Nach Beendigung der Zugabe wurde die kalte Mischung fünfzehn Minuten gerührt. Dann wurden rasch 56,8 g (230 mMol) 2 - Aethoxy - 1 - äthoxycarbonyl - 1,2 - dihydrochinolin in 100 ml Methylenchlorid zugefügt. Die Mischung wurde in der Kälte zwei Stunden und dann bei Raumtemperatur über Nacht gerührt, dann mit Wasser (100 ml) und schliesslich mit 1N Salzsäure (4×100 ml) gewaschen. Die kombinierten sauren Waschwässer wurden mit Methylenchlorid (2×50 ml) zurückextrahiert. Die vereinigten organischen Lösungen wurden mit Wasser (100 ml) und schliesslich mit Kaliumbicarbonatlösung (3×100 ml) gewaschen, dann über wasserfreiem Natriumsulfat getrocknet, filtriert und eingedampft. Das verbleibende Oel wurde mit Benzol nochmals eingedampft, was ein Oel ergab, welches mit wasserfreiem Aether (200 ml) verrieben wurde. Man erhielt so 58,2 g Rohprodukt vom Schmelzpunkt 77—80°C. Umkristallisation aus 200 ml Aethylacetat ergab 50,6 g Dimethyl - N - benzyloxycarbonyl - L - norvalyl - aminomethylphosphonat vom Schmelzpunkt 82—84°C.

29.1.2.1 Analog zu Beispiel 5.1.3 erhielt man aus dem Monobenzylaminsalz von (N - Benzyloxycarbonyl - L - norvalyl) - amino - methylphosphonsäure die (L - Norvalylamino) - methylphosphonsäure vom Schmelzpunkt 273—275°C (Zers.); $[\alpha]_D^{20}$=+61,2°; $[\alpha]_{365}^{20}$=+224° (c=0,54% in Wasser).

29.1.2.2 37,2 g (100 mMol) Dimethyl - N - benzyloxycarbonyl - L - norvalyl - aminomethylphosphonat wurden fünf Stunden in 120 ml 45%igem Bromwasserstoff in Eisessig gerührt. 500 ml Diäthyläther wurden dann zugefügt, worauf sich ein Oel ausschied, welches in 300 ml Methanol aufgenommen wurde. Die Lösung wurde gerührt und mit 40 ml Propylenoxid versetzt. Nach ungefähr fünf Minuten begann das Produkt auszukristallisieren. Die Mischung wurde über Nacht im Kühlschrank stehen gelassen. Der Festkörper wurde dann abfiltriert und aus einer Mischung von 200 ml heissem Wasser und 400 ml Aethanol umkristallisiert, was 16,7 g (L - Norvalylamino) - methylphosphonsäure vom Schmelzpunkt 293—294°C (Zers.) ergab; $[\alpha]_D^{20}$=+62,7° (c=0,5% in Wasser).

29.1.3 Analog zu Beispiel 1.2.1 erhielt man aus N - Benzyloxycarbonyl - L - norvalin - N - hydroxysuccinimid - ester und (L - norvalylamino) - methylphosphonsäure, nach Verreiben des Rückstands aus der Eindampfung aus dem sauren Eluat mit Aether, [(N - Benzyloxycarbonyl - L - norvalyl - L - norvalyl) - amino] - methylphosphonsäure vom Schmelzpunkt 175—185°C (Zers.).

29.1.4 Analog zu Beispiel 5.1.3 erhielt man aus [(N - Benzyloxycarbonyl - L - norvalyl - L - norvalyl)amino] - methylphosphonsäure die (L - Norvalyl - L - norvalylamino) - methylphosphonsäure vom Schmelzpunkt 265—267°C (Zers.); $[\alpha]_D^{20}$=—12,4°; $[\alpha]_{365}^{20}$=—36,4° (c=0,51% in 1N Salzsäure).

29.2 Das Verfahren

29.2.1 Analog zu Beispiel 1.2.1 erhielt man aus (L - Norvalyl - L - norvalylamino) - methylphosphonsäure und einer Lösung von N - Benzyloxycarbonyl - sarcosin - N - hydroxysuccinimid - ester, nach Behandlung des Rückstandes der Verdampfung des sauren Eluats mit Aether, [(N - Benzyloxycarbonyl - sarcosyl - L - norvalyl - L - norvalyl)amino] - methylphosphonsäure vom Schmelzpunkt 205—208°C (Zers.).

29.2.2 Analog zu Beispiel 5.1.3 erhielt man aus [(N - Benzyloxycarbonyl - sarcosyl - L - norvalyl - L - norvalyl)amino] - methylphosphonsäure die (Sarcosyl - L - norvalyl - L - norvalylamino) - methylphosphonsäure vom Schmelzpunkt 271—273°C (Zers.); $[\alpha]_D^{20}$=—56,2°; $[\alpha]_{365}^{20}$=—198° (c=0,55% in Wasser).

Beispiel 30

30.1 Analog zu Beispiel 1.2.1 erhielt man aus der Lösung von N - Benzyloxycarbonyl - N - methyl - L - valin - N - hydroxysuccinimid - ester in Dimethylformamid und (1R) - 1 - (L - Norvalyl - L - norvalylamino) - äthylphosphonsäure das Monobenzylaminsalz von (1R) - 1 - [(N - Benzyloxycarbonyl - N - methyl - L - valyl - L - norvalyl - L - norvalyl)amino] - äthylphosphonsäure vom Schmelzpunkt 217—227°C (Zers.).

30.2 Analog zu Beispiel 5.1.3 erhielt man aus dem Monobenzylaminsalz von (1R) - 1 - [(N - Benzyloxycarbonyl - N - methyl - L - valyl - L - norvalyl - L - norvalyl) - amino] - äthylphosphonsäure die (1R) - 1 - (N - Methyl - L - valyl - L - norvalyl - L - norvalylamino) - äthylphosphonsäure vom Schmelzpunkt 300—302°C (Zers.).

Beispiel 31

31.1 Herstellung des Ausgangsmaterials

31.1.1 Analog zu Beispiel 29.1.1.2 erhielt man aus N - Benzyloxycarbonyl - L - tyrosin und Dimethyl - (1R) - (L - alanylamino)äthylphosphonat, nach Umkristallisation aus Methanol, das Monomethanolat von Dimethyl - (1R) - 1 - [(N - benzyloxycarbonyl - L - tyrosyl - L - alanyl)amino] - äthylphosphonat vom Schmelzpunkt 103—106°C.

31.1.2 Das Monomethanolat von Dimethyl - (1R) - 1 - [(N - Benzyloxycarbonyl - L - tyrosyl -

L - alanyl)amino] - äthylphosphonat wurde in Methanol in Gegenwart von 10% Palladiumkohle-Katalysator hydriert. Der Katalysator wurde abfiltriert und das Filtrat eingedampft. Der Rückstand wurde nochmals mit Toluol eingedampft und dann in Aethanol/Chloroform suspendiert. Die Suspension wurde mit N - Benzyloxycarbonyl - sarcosin behandelt und die resultierende Mischung bei Raumtemperatur gerührt, während Triäthylamin zugefügt wurde. Nach fünfzehn Minuten wurde 2 - Aethoxy - 1 - äthoxycarbonyl - 1,2 - dihydrochinolin in Chloroform zugefügt. Die Mischung wurde bei Raumtemperatur über Nacht gerührt und dann eingedampft. Der Rückstand wurde mit Wasser gerührt, abfiltriert, gewaschen und getrocknet. Nach Umkristallisation aus Methanol/Diäthyläther erhielt man Dimethyl - (1R) - 1 - [(N - benzyloxycarbonyl - sarcosyl - L - tyrosyl - L - alanyl)amino] - äthylphosphonat.

31.2 Das Verfahren

31.2.1 Das gem. 31.1.2 erhaltene Phosphonat wurde bei Raumtemperatur 18 Stunden in Salzsäure/Essigsäure gerührt. Die nach Eindampfen erhaltene Festsubstanz wurde mit Aether behandelt und getrocknet. Die Festsubstanz wurde dann in Methanol aufgenommen, worauf Anilin zugefügt und die Mischung eine Stunde gerührt wurde. Das Anilinsalz von (1R) - 1 - [(N - Benzyloxycarbonyl - sarcosyl - L - tyrosyl - L - alanyl) - amino] - äthylphosphonsäure wurde abfiltriert, mit Methanol und dann mit Aether gewaschen und schliesslich getrocknet. Schmelzpunkt 210—214°C (Zers.).

31.2.2 Das erhaltene Anilinsalz wurde in Essigsäure suspendiert. Dann wurde 10% Palladiumkohle-Katalysator unter Argon zugefügt. Nach Zugabe von Essigsäure wurde die Mischung über Nacht bei Raumtemperatur gerührt und dann hydriert bis die Aufnahme des Wasserstoffs aufhörte. Der Katalysator wurde abfiltriert und das Filtrat eingedampft. Die verbleibende Festsubstanz wurde mit Methanol verrieben, abfiltriert, gewaschen und getrocknet. Dann wurde sie durch Fällung aus wässrigem HCl/Aethanol mit Propylenoxid gereinigt. Mann erhielt so (1R) - 1 - Sarcosyl - L - tyrosyl - L - alanylamino) - äthylphosphonsäure vom Schmelzpunkt 275—277°C (Zers.).

Beispiel 32

32.1 Herstellung des Ausgangsmaterials

32.1.1 N - Benzylglycin - hydrochlorid wurde in 2N Natriumhydroxidlösung aufgenommen und die Mischung bei —5°C gerührt, während 4N Natriumhydroxidlösung und Benzylchlorformiat gleichzeitig tropfenweise zugefügt wurden. Die Mischung wurde in der Kälte zwei Stunden gerührt und dann noch eine halbe Stunde bei Raumtemperatur. Ueberflüssiges Benzylchlorformiat wurde durch Extraktion mit Aether entfernt. Die wässrige Schicht wurde auf —5°C gekühlt und mit konzentrierter Salzsäure angesäuert. Die Mischung wurde dann in der Kälte eine Stunde gerührt, dann auf Raumptemperatur gebracht und vier Mal mit Diäthyläther extrahiert. Die Diäthylätherextrakte wurden mit Wasser gewaschen, filtriert und dann über wasserfreiem Natriumsulfat getrocknet und nochmals filtriert. Eindampfung ergab N - Benzyl - N - benzyloxycarbonyl - glycin in Form eines Oels.

32.1.2 Analog zu Beispiel 29.1.1.2 erhielt man aus N - Benzyloxycarbonyl - L - alanin und Dimethyl - (1R) - 1 - (L - alanylamino) - äthylphosphonat - hydrochlorid das Dimethyl - (1R) - 1 - [(N - Benzyloxycarbonyl - L - alanyl - L - alanyl)amino] - äthylphosphonat vom Schmelzpunkt 153—155°C.

32.1.3 Das erhaltene Phosphonat wurde in Methanol gelöst und in Gegenwart von 10% Palladiumkohle-Katalysator katalytisch hydriert. Die Lösung wurde filtriert und eingedampft. Man erhielt (1R) - 1 - (L - Alanyl - L - alanylamino) - äthylphosphonat in Form eines Oels.

32.2 Das Verfahren

32.2.1 Das gemäss 32.1.3 erhaltene Phosphonat wurde in Methylenchlorid gelöst und die Lösung mit N - Benzyl - N - benzyloxycarbonyl - glycin behandelt. Die erhaltene Mischung wurde gerührt, auf —10°C abgekühlt, worauf 2 - Aethoxy - 1 - äthoxycarbonyl - 1,2 - dihydrochinolin rasch zugefügt wurde. Die Mischung wurde in der Kälte zwei Stunden gerührt und dann noch sechzehn Stunden bei Raumtemperatur. Nach Aufarbeitung in üblicher Weise erhielt man Dimethyl - (1R) - 1 - [(N - benzyl - N - benzyloxycarbonyl - glycyl - L - alanyl - L - alanyl) - amino] - äthylphosphonat vom Schmelzpunkt 114—117°C.

32.2.2 Analog zu Beispiel 5.1.3 erhielt man aus Dimethyl - (1R) - 1 - [(N - Benzyl - N - benzyloxycarbonyl - glycyl - L - alanyl - L - alanyl)amino] - äthylphosphonat die (1R) - 1 - (N - Benzyl - glycyl - L - alanyl - L - alanylamino) - äthylphosphonsäure vom Schmelzpunkt 295—296°C (Zers.).

Beispiel 33

33.1 Herstellung des Ausgangsmaterials

33.1.1 Zu einer auf 5°C gekühlten Lösung von Cyclopropylamin in Diäthyläther wurde Aethylbromacetat in Diäthyläther tropfenweise zugefügt. Es bildete sich eine weise Festsubstanz. Das Gemisch wurde drei Stunden unter Kühlen gerührt. Der Niederschlag wurde abfiltriert, mit Aether extrahiert und das Filtrat eingedampft. Der Rückstand wurde mit Benzol/Cyclohexan verrieben und die Festsubstanz

abfiltriert. Das Filtrat wurde eingedampft und der Rückstand destilliert und fraktioniert. Man erhielt so Aethyl-cyclopropylaminoacetat vom Siedepunkt 32—33°C/0,7 mmHg; $n_D^{19}$=1,4384.

33.1.2 Aethyl-cyclopropylaminoacetat wurde in einer Mischung von konzentrierter Salzsäure und Wasser bis zum Nachlassen der Reaktion gerührt. Das Gemisch wurde dann zwei Stunden unter Rückfluss erhitzt. Die resultierende Lösung wurde eingedampft, die weisse Festsubstanz mit Aceton verrieben und aus Methanol/Aethylacetat umkristallisiert. Man erhielt so Cyclopropylaminoessigsäure-hydrochlorid vom Schmelzpunkt 189—191°C (Zers.).

33.1.3 Analog zu Beispiel 32.1.1 erhielt man aus Cyclopropylaminoessigsäure-hydrochlorid und Benzylchlorformiat das N - Benzyloxycarbonyl - N - cyclopropyl - glycin in Form eines Oels, welches schliesslich fest wurde. Diese Substanz wurde im nächsten Schritt ohne weitere Reinigung verwendet.

33.2 Das Verfahren

33.2.1 Analog zu Beispiel 29.1.1.2 erhielt man aus Dimethyl - (1R) - (L - alanyl - L - alanylamino) - äthylphosphonat (hergestellt gemäss Beispiel 32.1.2) und N - Benzyloxycarbonyl - N - cyclopropyl - glycin das Dimethyl - N - (1R) - 1 - [(N - benzyloxycarbonyl - N - cyclopropyl - glycyl - L - alanyl - L - alanyl)amino] - äthylphosphonat vom Schmelzpunkt 120—123°C.

33.2.2 Dieses wurde gemäss Beispiel 5.1.3 in (1R) - 1 - (N - Cyclopropyl - glycyl - L - alanyl - L - alanylamino) - äthylphosphonsäure vom Schmelzpunkt 287—288°C (Zers.) übergeführt.

Beispiel 34

34.1 Herstellung des Ausgangsmaterials

Analog zu Beispiel 32.1.1 erhielt man aus tert. - Butylaminoessigsäure - hydrochlorid und Benzylchlorformiat das N - Benzyloxycarbonyl - N - tert.butyl - glycin in Form eines Oels.

34.2 Das Verfahren

34.2.1 Analog zu Beispiel 32.2.1 erhielt man aus Dimethyl - (1R) - 1 - (L - alanyl - L - alanylamino) - äthylphosphonsäure und N - Benzyloxycarbonyl - N - tert.butyl - glycin das Dimethyl - (1R) - 1 - [(N - benzyloxycarbonyl - N - tert.butyl - glycyl - L - alanyl - L - alanyl)amino] - äthylphosphonat vom Schmelzpunkt 75—90°C.

34.2.2 Dieses wurde gemäss Beispiel 32.1.3 in Dimethyl - (1R) - 1 - [(N - tert.butyl - glycyl - L - alanyl - L - alanyl)amino] - äthylphosphonat vom Schmelzpunkt 148—152°C übergeführt.

34.2.3 Aus diesem erhielt man analog zu Beispiel 5.1.3 die (1R) - 1 - (N - tert.Butyl - glycyl - L - alanyl - L - alanylamino) - äthylphosphonsäure vom Schmelzpunkt 272—274°C (Zers.).

Beispiel 35

35.1 Analog zu Beispiel 32.2.1 erhielt man aus Dimethyl - (1R) - 1 - (L - alanyl - L - alanylamino) - äthylphosphonat und N - Benzyloxycarbonyl - N - phenyl - glycin (hergestellt gemäss J. Med. Chem., *15*, (1972), 720—726) Dimethyl - (1R) - 1 - [(N - benzyloxycarbonyl - N - phenyl - glycyl - L - alanyl - L - alanyl)amino] - äthylphosphonat vom Schmelzpunkt 123—125°C.

35.2 Aus diesem wurde analog zu Beispiel 5.1.3 die (1R) - 1 - (N - Phenyl - glycyl - L - alanyl - L - alanylamino) - äthylphosphonsäure vom Schmelzpunkt 216—218°C (Zers.) erhalten.

Beispiel A

Eine 1000 ml Injektionslösung wurde wie folgt hergestellt:

| | Per 1000 ml |
|---|---|
| (1R)-1-(Sarcosyl-glycyl-L-alanylamino)-äthylphosphonsäure | 50,0 g |
| Chlorcresol | 1,0 g |
| Eisessig | 1,2 g |
| Natriumhydroxidlösung (0,1N) *q.s.* | ad pH 4,5 |
| Wasser für Injektionszwecke | ad 1000 ml |

**Patentansprüche**

1. Phosphorhaltige Peptidderivate der allgemeinen Formel

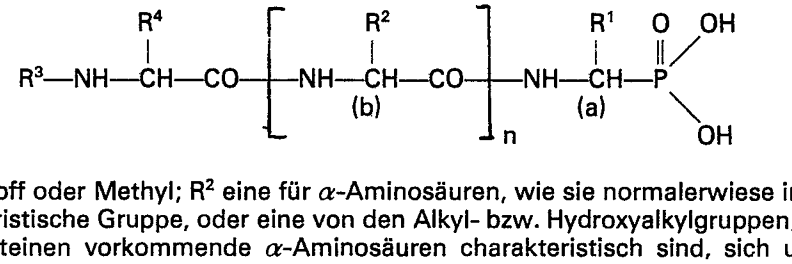

(I)

worin $R^1$ Wasserstoff oder Methyl; $R^2$ eine für $\alpha$-Aminosäuren, wie sie normalerweise in Proteinen vorkommen, charakteristische Gruppe, oder eine von den Alkyl- bzw. Hydroxyalkylgruppen, wie sie für normalerweise in Proteinen vorkommende $\alpha$-Aminosäuren charakteristisch sind, sich unterscheidende

$C_2$—$C_8$-Alkyl- bzw. $C_2$—$C_8$-Hydroxyalkylgruppe; $R^3$ $C_1$—$C_8$-Alkyl, $C_3$—$C_8$-Cycloalkyl oder Allyl und $R^4$ Wasserstoff oder $C_1$—$C_8$-Alkyl bedeuten; n für 2 oder 3 steht; mit R-Konfiguration an mit (a) bezeichneten C-Atom (falls $R^1 \neq H$) und L-Konfiguration am mit (b) bezeichneten C-Atom (falls $R^2 \neq H$); sowie pharmazeutisch verträgliche Salze solcher Verbindungen.

2. Peptidderivate nach Anspruch 1, worin $R^3$ $C_1$—$C_8$-Alkyl oder Allyl, $R^4$ Wasserstoff und n=2 bedeuten.

3. Peptidderivate nach Anspruch 1 oder 2, worin $R^1$ Methyl bedeutet.

4. Peptidderivate nach den Ansprüchen 1—3, worin $R^2$ eine für $\alpha$-Aminosäuren, wie sie normalerweise in Proteinen vorkommen, charakteristische Gruppe oder eine sich von solchen charakteristischen Gruppen unterscheidende $C_2$—$C_8$-Alkylgruppe bedeutet.

5. Peptidderivate nach den Ansprüchen 1—4, worin $R^2$ $C_1$—$C_8$-Alkyl bedeutet.

6. Peptidderivate nach Anspruch 5, worin $R^3$ Methyl bedeutet.

7. (1R) - 1 - (Sarcosyl - L - alanyl - L - alanylamino) - äthylphosphonsäure oder ein pharmazeutisch verträgliches Salz davon.

8. (1R) - 1 - (Sarcosyl - L - alanyl - L - arginylamino) - äthylphosphonsäure oder ein pharmazeutisch verträgliches Salz davon.

9. (1R) - 1 - (Sarcosyl - glycyl - L - norvalyl - L - norvalylamino) - äthylphosphonsäure oder ein pharmazeutisch verträglisches Salz davon.

10. (1R) - 1 - (N - Methyl - L - norvalyl - L - norvalyl - L - norvalylamino) - äthylphosphonsäure oder ein pharmazeutisch verträgliches Salz davon.

11. (1R) - 1 - (Sarcosyl - L - valyl - L - valyl - L - norvalylamino) - äthylphosphonsäure oder ein pharmazeutisch verträgliches Salz davon.

12. (1R) - 1 - (Sarcosyl - L - norvalyl - L - norvalylamino) - äthylphosphonsäure oder ein pharmazeutisch verträgliches Salz davon.

13. (1R) - 1 - (Sarcosyl - L - methionyl - L - alanylamino) - äthylphosphonsäure oder ein pharmazeutisch verträgliches Salz davon.

14. (1R) - 1 - (N - Allyl - glycyl - L - alanyl - L - alanylamino) - äthylphosphonsäure oder ein pharmazeutisch verträgliches Salz davon.

15. (1R) - 1 - (Sarcosyl - L - histidyl - L - alanylamino) - äthylphosphonsäure oder ein pharmazeutisch verträgliches Salt davon.

16. (Sarcosyl - L - alanyl - L - alanylamino) - methylphosphonsäure oder ein pharmazeutisch verträgliches Salz davon.

17. (Sarcosyl - L - norvalyl - L - norvalylamino) - methylphosphonsäure oder ein pharmazeutisch verträgliches Salz davon.

18. (1R) - 1 - (N - Aethyl - glycyl - L - alanyl - L - alanylamino) - äthylphosphonsäure oder ein pharmazeutisch verträgliches Salz davon.

19. (1R) - 1 - (N - (n - Propyl) - glycyl - L - alanyl - L - alanylamino) - äthylphosphonsäure oder ein pharmazeutisch verträgliches Salz davon.

20. (1R) - 1 - (N - (n - Hexyl) - glycyl - L - alanyl - L - alanylamino) - äthylphosphonsäure oder ein pharmazeutisch verträgliches Salz davon.

21. (1R) - 1 - (N - Methyl - L - valyl - L - valyl - L - norvalylamino) - äthylphosphonsäure oder ein pharmazeutisch verträgliches Salt davon.

22. (1R) - 1 - (N - Methyl - L - leucyl - L - norvalyl - L - norvalylamino) - äthylphosphonsäure oder ein pharmazeutisch verträgliches Salz davon.

23. (1R) - 1 - (N - Methyl - L - valyl - L - norvalyl - L - norvalylamino) - äthylphosphonsäure oder ein pharmazeutisch verträgliches Salz davon.

24. (1R) - 1 - (Sarcosyl - L - tyrosyl - L - alanylamino) - äthylphosphonsäure oder ein pharmazeutisch verträgliches Salz davon.

25. (1R) - 1 - (Sarcosyl - L - alanyl - L - serylamino) - äthylphosphonsäure oder ein pharmazeutisch verträgliches Salz davon.

26. (1R) - 1 - (N - Cyclopropyl - glycyl - L - alanyl - L - alanylamino) - äthylphosphonsäure oder ein pharmazeutisch verträgliches Salz davon.

27. (1R) - 1 - (N - tert.Butyl - glycyl - L - alanyl - L - alanylamino) - äthylphosphonsäure oder ein pharmazeutisch verträgliches Salz davon.

28. Verbindungen der allgemeinen Formel

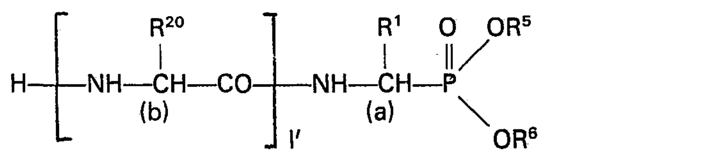

(IIa)

worin l' für 2 oder 3 steht; $R^5$ und $R^6$ Wasserstoff oder eine $C_1$—$C_8$-Alkylgruppe bedeuten; $R^1$ wie in Anspruch 1 definiert ist; $R^{20}$ dasselbe wie $R^2$ bedeutet, wobei jede Aminogruppe geschützt sein kann und wobei mindestens ein $R^{20}$ eine $C_2$—$C_8$-Alkyl- bzw. $C_2$—$C_8$-Hydroxyalkylgruppe darstellt, die sich von

den Alkyl- bzw. Hydroxyalkylgruppen, wie sie für normalerweise in Proteinen vorkommende $\alpha$-Aminosäuren charakteristisch sind, unterscheidet oder solch eine $C_2$—$C_8$-Hydroxyalkylgruppe in geschützer Form; und worin (a) und (b) dasselbe wie oben bedeuten, wenn sie zur Herstellung von Verbindungen gemäss den Ansprüchen 1—27 verwendet werden.

29. Verbindungen der allgemeinen Formel

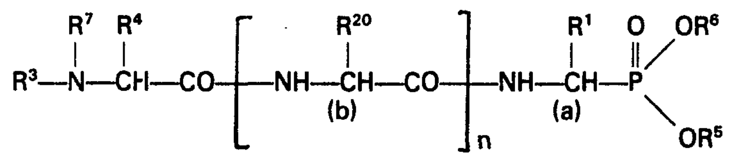

(Ia)

worin $R^7$ eine Aminoschutzgruppe darstellt und die übrigen Symbole dasselbe wie in Anspruch 1 bzw. 28 bedeuten, wenn sie zur Herstellung von Verbindungen gemäss den Ansprüchen 1—27 verwendet werdon.

30. Peptidderivate nach den Ansprüchen 1—27 als pharmazeutische, insbesondere antibakterielle Wirkstoffe.

31. Verfahren zur Herstellung von Verbindungen der allgemeinen Formel I nach Anspruch 1 oder 2, dadurch gekennzeichnet, dass man a) die Schutzgruppe(n) aus einer Verbindung der allgemeinen Formel

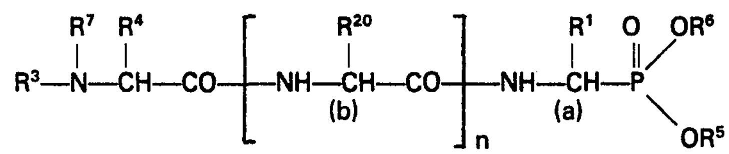

(Ia)

worin n, $R^1$, $R^3$ und $R^4$ dasselbe wie oben, $R^5$ und $R^6$ Wasserstoff oder eine Niederalkyl-Schutzgruppe bedeuten; $R^7$ eine Aminoschutzgruppe und $R^{20}$ dasselbe wie $R^2$ bedeutet, wobei jede Aminogruppe geschützt sein kann und gegebenenfalls anwesende andere funktionelle Gruppen erforderlichenfalls geschützt sein können; und worin (a) und (b) dasselbe wie in Anspruch 1 bedeuten, nach an sich bekannten Methoden abspaltet und b) eine erhaltene Verbindung der allgemeinen Formel I gewünschtenfalls in ein pharmazeutisch verträgliches Salz überführt.

32. Arzneimittel, enthaltend eine Verbindung der allgemeinen Formel I gemäss Anspruch 1 oder ein pharmazeutisch verträgliches Salz einer solchen Verbindung.

33. Antibakterielles Mittel, enthaltend eine Verbindung der allgemeinen Formel I gemäss Anspruch 1 oder ein pharmazeutisch verträgliches Salz einer solchen Verbindung.

**Claims**

1. Phosphorus containing peptide derivatives of the general formula

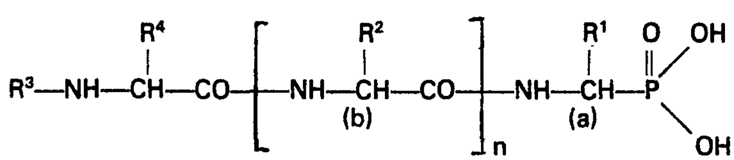

(I)

wherein $R^1$ represents hydrogen or methyl; $R^2$ represents the characterising group of $\alpha$-amino acids of the type normally found in proteins or a $C_2$—$C_8$-alkyl or $C_2$—$C_8$-hydroxyalkyl group other than the alkyl or hydroxyalkyl characterising groups of $\alpha$-amino acids of the type normally found in proteins; $R^3$ represents $C_1$—$C_8$-alkyl, $C_3$—$C_6$-cycloalkyl or allyl and $R^4$ represents hydrogen or $C_1$—$C_8$-alkyl; n stands for 2 and 3; with R-configuration at the C-atom designated as (a) (when $R^1 \neq H$) and L-configuration at the C-atom designated as (b) (when $R^2 \neq H$), as well as pharmaceutically acceptable salts of such compounds.

2. Peptide derivatives according to claim 1, wherein $R^3$ represents $C_1$—$C_8$-alkyl or allyl, $R^4$ represents hydrogen and n=2.

3. Peptide derivatives according to claim 1 or 2, wherein $R^1$ represents methyl.

4. Peptide derivatives according to claims 1—3, wherein $R^2$ represents the characterising group of $\alpha$-amino acids of the type normally found in proteins or a $C_2$—$C_8$-alkyl group other than such characterising groups.

5. Peptide derivatives according to claims 1—4, wherein $R^2$ represents $C_1$—$C_8$-alkyl.

6. Peptide derivatives according to claim 5, wherein $R^3$ represents methyl.

7. (1R) - 1 - (Sarcosyl - L - alanyl - L - alanylamino) - ethylphosphonic acid or a pharmaceutically acceptable salt thereof.

8. (1R) - 1 - (Sarcosyl - L - alanyl - L - arginylamino) - ethylphosphonic acid or a pharmaceutically acceptable salt thereof.

9. (1R) - 1 - (Sarcosyl - glycyl - L - norvalyl - L - norvalylamino) - ethylphosphonic acid or a pharmaceutically acceptable salt thereof.

10. (1R) - 1 - (N - Methyl - L - norvalyl - L - norvalyl - L - norvalylamino) - ethylphosphonic acid or a pharmaceutically acceptable salt thereof.

11. (1R) - 1 - (Sarcosyl - L - valyl - L - valyl - L - norvalylamino) - ethylphosphonic acid or a pharmaceutically acceptable salt thereof.

12. (1R) - 1 - (Sarcosyl - L - norvalyl - L - norvalylamino) - ethylphosphonic acid or a pharmaceutically acceptable salt thereof.

13. (1R) - 1 - (Sarcosyl - L - methionyl - L - alanylamino) - ethylphosphonic acid or a pharmaceutically acceptable salt thereof.

14. (1R) - 1 - (N - Allyl - glycyl - L - alanyl - L - alanylamino) - ethylphosphonic acid or a pharmaceutically acceptable salt thereof.

15. (1R) - 1 - (Sarcosyl - L - histidyl - L - alanylamino) - ethylphosphonic acid or a pharmaceutically acceptable salt thereof.

16. (Sarcosyl - L - alanyl - L - alanylamino) - methylphosphonic acid or a pharmaceutically acceptable salt thereof.

17. (Sarcosyl - L - norvalyl - L - norvalylamino) - methylphosphonic acid or a pharmaceutically acceptable salt thereof.

18. (1R) - 1 - (N - Ethyl - glycyl - L - alanyl - L - alanylamino) - ethylphosphonic acid or a pharmaceutically acceptable salt thereof.

19. (1R) - 1 - (N - (n - Propyl) - glycyl - L - alanyl - L - alanylamino) - ethylphosphonic acid or a pharmaceutically acceptable salt thereof.

20. (1R) - 1 - (N - (n - Hexyl) - glycyl - L - alanyl - L - alanylamino) - ethylphosphonic acid or a pharmaceutically acceptable salt thereof.

21. (1R) - 1 - (N - Methyl - L - valyl - L - valyl - L - norvalylamino) - ethylphosphonic acid or a pharmaceutically acceptable salt thereof.

22. (1R) - 1 - (N - Methyl - L - leucyl - L - norvalyl - L - norvalylamino) - ethylphosphonic acid or a pharmaceutically acceptable salt thereof.

23. (1R) - 1 - (N - Methyl - L - valyl - L - norvalyl - L - norvalylamino) - ethylphosphonic acid or a pharmaceutically acceptable salt thereof.

24. (1R) - 1 - (Sarcosyl - L - tyrosyl - L - alanylamino) - ethylphosphonic acid or a pharmaceutically acceptable salt thereof.

25. (1R) - 1 - (Sarcosyl - L - alanyl - L - serylamino) - ethylphosphonic acid or a pharmaceutically acceptable salt thereof.

26. (1R) - 1 - (N - Cyclopropyl - glycyl - L - alanyl - L - alanylamino) - ethylphosphonic acid or a pharmaceutically acceptable salt thereof.

27. (1R) - 1 - (N - tert.Butyl - glycyl - L - alanyl - L - alanylamino) - ethylphosphonic acid or a pharmaceutically acceptable salt thereof.

28. Compounds of the general formula

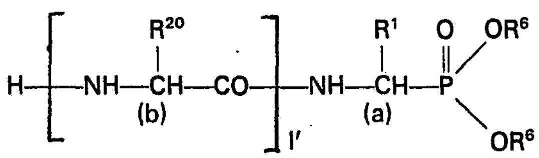

(IIa)

wherein l′ stands for 2 or 3; $R^5$ and $R^6$ represent hydrogen or a $C_1$—$C_8$-alkyl group; $R^1$ is as defined in claim 1; $R^{20}$ has the same meaning as $R^2$, whereby each amino group may be protected and whereby at least one $R^{20}$ represents a $C_2$—$C_8$-alkyl or $C_2$—$C_8$-hydroxyalkyl group other than the characterising alkyl or hydroxyalkyl groups of $\alpha$-amino acids normally found in proteins or such a $C_2$—$C_8$-hydroxyalkyl group in protected form; and (a) and (b) have the same meanings as given above whenever used for the preparation of compounds according to claims 1 to 27.

29. Compounds of the general formula

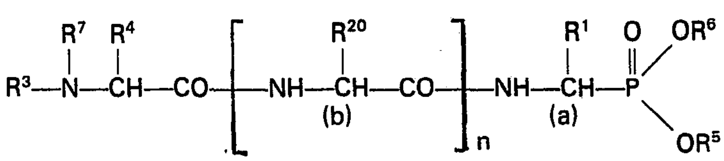

(Ia)

wherein $R^7$ represents an amino protecting group and the other symbols have the same meanings given in claims 1 or 28 whenever used for the preparation of compounds according to claims 1 to 27.

30. Peptide derivatives according to claims 1 to 27 as pharmaceutically, in particular antibacterially active substances.

31. A process for the preparation of compounds of the general formula I according to claim 1 or 2, characterised in that one

(a) cleaves off by known methods the protecting group(s) from a compound of the general formula

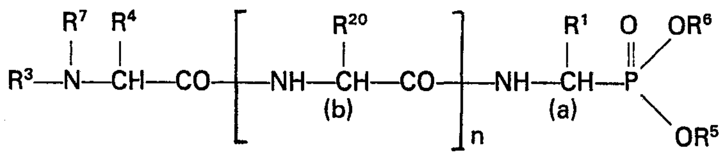

(Ia)

wherein n, $R^1$, $R^3$ and $R^4$ are as defined above; $R^5$ and $R^6$ represent hydrogen or a lower alkyl protecting group; $R^7$ represents an amino protecting group; $R^{20}$ has the same meaning as $R^2$, whereby each amino group may be in protected form and any other functional groups which may be present, are if necessary protected and wherein (a) and (b) have the same meanings as in claim 1 and

(b) if desired, converts a compound of general formula I obtained into a pharmaceutically acceptable salt.

32. Medicament containing a compound of the general formula I according to claim 1 or a pharmaceutically acceptable salt of such a compound.

33. Anti-bacterial agent containing a compound of the general formula I according to claim 1 or a pharmaceutically acceptable salt of such a compound.

**Revendications**

1. Dérivés peptidiques contenant du phosphore, de formule générale

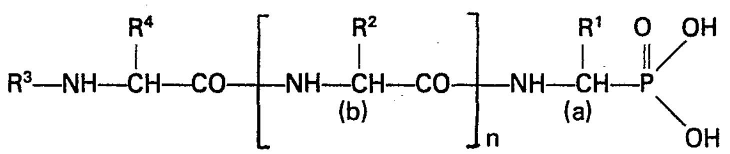

(I)

où $R^1$ représente un hydrogène ou un méthyle, $R^2$ représente un groupe caractéristique des acides $\alpha$-aminés que l'on trouve normalement dans les protéines, ou un groupe alcoyle en $C_2$ à $C_8$ ou hydroxyalcoyle en $C_2$ à $C_8$ se différenciant des groupes alcoyle ou hydroxyalcoyle caractéristiques des acides $\alpha$-aminés qui l'on trouve normalement dans les protéines; $R^3$ représente un alcoyle en $C_1$ à $C_8$, un cycloalcoyle en $C_3$ à $C_6$ ou un allyle et $R^4$ représente un hydrogène ou un alcoyle en $C_1$ à $C_8$; n vaut 2 ou 3; avec une configuration R sur l'atome de carbone désigné par (a) (si $R^1 \neq H$) et une configuration L sur l'atome de carbone désigné par (b) (si $R^2 \neq H$); ainsi que les sels pharmaceutiquement acceptables de tels composés.

2. Dérivés peptidiques selon la revendication 1, où $R^3$ représénte un alcoyle en $C_1$ à $C_8$ ou un allyle, $R^4$ représente un hydrogène et n=2.

3. Dérivés peptidiques selon les revendications 1 ou 2, où $R^1$ représente un méthyle.

4. Dérivés peptidiques selon les revendications 1—3, où $R^2$ représente un groupe caractéristique des acides $\alpha$-aminés que l'on trouve normalement dans les protéines ou un groupe alcoyle en $C_2$ à $C_8$ se différenciant de tels groupes caractéristiques.

5. Dérivés peptidiques selon les revendications 1—4, où $R^2$ représente un alcoyle en $C_1$ à $C_8$.

6. Dérivés peptidiques selon la revendication 5, où $R^3$ représente un méthyle.

7. Acide (1R) - 1 - (sarcosyl - L - alanyl - L - alanylamino) - éthylphosphonique ou un de ses sels pharmaceutiquement acceptables.

8. Acide (1R) - 1 - (sarcosyl - L - alanyl - L - arginylamino) - éthylphosphonique ou un de ses sels pharmaceutiquement acceptables.

9. Acide (1R) - 1 - (sarcosyl - glycyl - L - norvalyl - L - norvalylamino) - éthylphosphonique ou un de ses sels pharmaceutiquement acceptables.

10. Acide (1R) - 1 - (N - méthyl - L - norvalyl - L - norvalyl - L - norvalylamino) - éthylphosphonique ou un de ses sels pharmaceutiquement acceptables.

11. Acide (1R) - 1 - (sarcosyl - L - valyl - L - valyl - L - norvalylamino) - éthylphosphonique ou un de ses sels pharmaceutiquement acceptables.

12. Acide (1R) - 1 - (sarcosyl - L - norvalyl - L - norvalylamino) - éthylphosphonique ou un de ses sels pharmaceutiquement acceptables.

13. Acide (1R) - 1 - (sarcosyl - L - méthionyl - L - alanylamino) - éthylphosphonique ou un de ses sels pharmaceutiquement acceptables.

14. Acide (1R) - 1 - (N - allyl - glycyl - L - alanyl - L - alanylamino) - éthylphosphonique ou un de ses sels pharmaceutiquement acceptables.

15. Acide (1R) - 1 - (sarcosyl - L - histidyl - L - alanylamino) - éthylphosphonique ou un de ses sels pharmaceutiquement acceptables.

16. Acide (sarcosyl - L - alanyl - L - alanylamino) - méthylphosphonique ou un de ses sels pharmaceutiquement acceptables.

17. Acide (sarcosyl - L - norvalyl - L - norvalylamino) - méthylphosphonique ou un de ses sels pharmaceutiquement acceptables.

18. Acide (1R) - 1 - (N - éthyl - glycyl - L - alanyl - L - alanylamino) - éthylphosphonique ou un de ses sels pharmaceutiquement acceptables.

19. Acide (1R) - 1 - (N - (n - propyl) - glycyl - L - alanyl - L - alanylamino) - éthylphosphonique ou un de ses sels pharmaceutiquement acceptables.

20. Acide (1R) - 1 - (N - (n - hexyl) - glycyl - L - alanyl - L - alanylamino) - éthylphosphonique ou un de ses sels pharmaceutiquement acceptables.

21. Acide (1R) - 1 - (N - méthyl - L - valyl - L - valyl - L - norvalylamino) - éthylphosphonique ou un de ses sels pharmaceutiquement acceptables.

22. Acide (1R) - 1 - (N - méthyl - L - leucyl - L - norvalyl - L - norvalylamino) - éthylphosphonique ou un de ses sels pharmaceutiquement acceptables.

23. Acide (1R) - 1 - (N - méthyl - L - valyl - L - norvalyl - L - norvalylamino) - éthylphosphonique ou un de ses sels pharmaceutiquement acceptables.

24. Acide (1R) - 1 - (sarcosyl - L - tyrosyl - L - alanylamino) - éthylphosphonique ou un de ses sels pharmaceutiquement acceptables.

25. Acide (1R) - 1 - (sarcosyl - L - alanyl - L - sérylamino) - éthylphosphonique ou un de ses sels pharmaceutiquement acceptables.

26. Acide (1R) - 1 - (N - cyclopropyl - glycyl - L - alanyl - L - alanylamino) - éthylphosphonique ou un de ses sels pharmaceutiquement acceptables.

27. Acide (1R) - 1 - (N - tert.butyl - glycyl - L - alanyl - L - alanylamino) - éthylphosphonique ou un de ses sels pharmaceutiquement acceptables.

28. Composés de formule générale

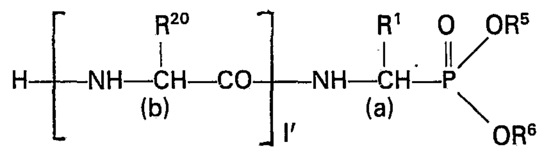

(IIa)

où l'vaut 2 ou 3; $R^5$ et $R^6$ représentent un hydrogène ou un groupe alcoyle en $C_1$ à $C_8$; $R^1$ est défini comme dans la revendication 1; $R^{20}$ a la même signification que $R^2$, où chaque groupe amino peut être protégé et où au moins un radical $R^{20}$ représente un groupe alcoyle en $C_2$ à $C_8$ ou hydroxyalcoyle en $C_2$ à $C_8$ qui se différencie des groupes alcoyle ou hydroxyalcoyle qui sont caractéristiques des acides $\alpha$-aminés que l'on trouve normalement dans les protéines, ou un tel groupe hydroxyalcoyle en $C_2$ à $C_8$ sous forme protégée; et où (a) et (b) ont la même signification que ci-dessus, lorsqu'on les utilise pour préparer des composés selon les revendications 1—27.

29. Composés de formule générale

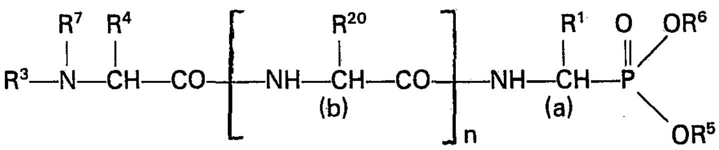

(Ia)

où $R^7$ représente un groupe protecteur d'amino et où les autres symboles ont la même signification que dans la revendication 1 ou la revendication 28, lorsqu'on les utilise pour préparer des composés selon les revendications 1—27.

30. Dérivés peptidiques selon les revendications 1 à 27 comme substances actives pharmaceutiques, en particulier anti-bactériennes.

31. Procédé de préparation de composés de formule générale I selon les revendications 1 ou 2, caractérisé en ce que

a) On sépare par des procédés connus le(s) groupe(s) protecteur(s) d'un composé de formule générale

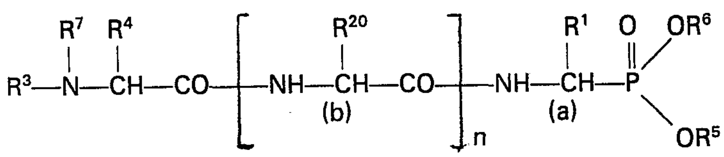

(Ia)

où n, $R^1$, $R^3$ et $R^4$ ont la même signification que ci-dessus, $R^5$ et $R^6$ représentent un hydrogène ou un groupe protecteur d'alcoyle inférieur; $R^7$ représente un groupe protecteur d'amino et $R^{20}$ a la même signification que $R^2$, où chaque groupe amino peut être protégé et où d'autres groupes fonctionnels éventuellement présents peuvent si nécessaire être protégés; et où (a) et (b) ont la même signification que dans la revendication 1, et

b) on transforme si on le désire un composé de formule générale I obtenu en un sel pharmaceutiquement acceptable.

32. Médicament contenant un composé de formule générale I selon la revendication 1 ou un sel pharmaceutiquement acceptable d'un tel composé.

33. Agent anti-bactérien contenant un composé de formule générale I selon la revendication 1 ou un sel pharmaceutiquement acceptable d'un tel composé.